(19) Europäisches Patentamt
European Patent Office
Office européen des brevets

(11) EP 3 601 219 B1

(12) FASCICULE DE BREVET EUROPEEN

(45) Date de publication et mention de la délivrance du brevet:
**05.05.2021 Bulletin 2021/18**

(21) Numéro de dépôt: **18714327.6**

(22) Date de dépôt: **19.03.2018**

(51) Int Cl.:
***C07C 303/44*** *(2006.01)* ***C07C 309/15*** *(2006.01)*

(86) Numéro de dépôt international:
**PCT/FR2018/050652**

(87) Numéro de publication internationale:
**WO 2018/172676 (27.09.2018 Gazette 2018/39)**

# (54) FORME CRISTALLINE HYDRATEE DE L'ACIDE 2-ACRYLAMIDO-2-METHYLPROPANE SULFONIQUE

KRISTALLINE, MIT 2-ACRYLAMIDO-2-METHYLPROPANSULFONSÄURE HYDRATISIERT FORM

CRYSTALLINE FORM HYDRATED WITH 2-ACRYLAMIDO-2-METHYLPROPANE SULPHONIC ACID

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.03.2017 FR 1752288**

(43) Date de publication de la demande:
**05.02.2020 Bulletin 2020/06**

(73) Titulaire: **SPCM SA**
**42160 Andrézieux-Bouthéon (FR)**

(72) Inventeurs:
- **FAVERO, Cédrick**
**42160 Andrezieux Boutheon (FR)**
- **KIEFFER, Johann**
**42160 Andrezieux Boutheon (FR)**

(74) Mandataire: **Cabinet Laurent & Charras**
**Le Contemporain**
**50 Chemin de la Bruyère**
**69574 Dardilly Cedex (FR)**

(56) Documents cités:
**JP-B2- 3 412 158 US-A1- 2010 274 048**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 601 219 B1

## Description

### DOMAINE DE L'INVENTION

Le domaine de l'invention concerne une forme cristalline de l'acide 2-acrylamido-2-méthylpropane sulfonique (ATBS). Plus précisément, la présente invention a pour objet une forme cristalline hydratée de l'acide 2-acrylamido-2-méthylpropane sulfonique. L'invention concerne aussi le procédé d'obtention de la forme cristalline hydratée de l'acide 2-acrylamido-2-méthylpropane sulfonique.

### ETAT ANTERIEUR DE LA TECHNIQUE

L'acide 2-acrylamido-2-méthylpropane sulfonique, également appelé ATBS, est largement utilisé comme additif dans les fibres acryliques, ou encore comme matière première pour obtenir des polymères utilisés en tant que dispersant, épaississant floculant ou superabsorbant dans divers secteurs comme l'industrie pétrolière, la construction, le textile, le traitement des eaux (dessalement de l'eau de mer, industrie minérale, etc...) ou la cosmétique.

La réaction mise en œuvre dans le procédé de préparation de l'acide 2-acrylamido-2-méthylpropane sulfonique répond au schéma réactionnel ci-dessous, dans lequel l'acrylonitrile est présent en excès de manière à être à la fois le solvant de la réaction et un réactif. L'acrylonitrile est mis en contact avec de l'acide sulfurique fumant (oléum) et de l'isobutylène.

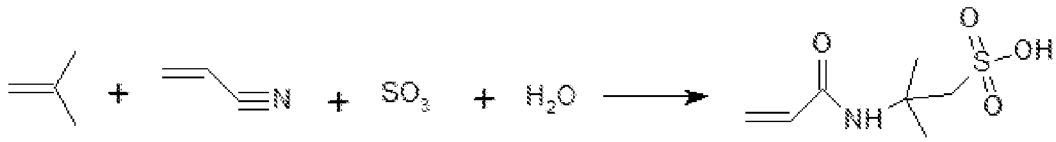

Un sous-produit pouvant être généré lors de cette synthèse est l'acrylamide.

L'acide 2-acrylamido-2-méthylpropane sulfonique n'est pas soluble dans le solvant acrylonitrile. Par conséquent le produit de réaction est sous une forme de suspension de cristaux dans le solvant de réaction.

A titre d'exemples, les documents US 6,448,347 et CN 102351744 décrivent un procédé de fabrication de l'acide 2-acrylamido-2-méthylpropane sulfonique en mode continu. L'acide 2-acrylamido-2-méthylpropane sulfonique est, par la suite, séparé de l'acrylonitrile, généralement par filtration, puis séché.

Le séchage de l'acide 2-acrylamido-2-méthylpropane sulfonique est nécessaire afin de diminuer la quantité d'acrylonitrile et d'acrylamide restant présent dans le cristal. Ces deux composés ont une classification comme cancérogène, mutagène ou toxique pour la reproduction (CMR). Il est donc nécessaire de procéder à une filtration efficace pour essorer au mieux l'acrylonitrile, et ensuite sécher l'acide 2-acrylamido-2-méthylpropane sulfonique afin d'obtenir des teneurs en acrylonitrile et acrylamide qui soient faibles.

Il est connu de l'homme de l'art que les cristaux de l'acide 2-acrylamido-2-méthylpropane sulfonique ont un arrangement cristallographique qui produit un solide de forme aiguille.

Les cristaux ayant une forme d'aiguille sont connus de l'homme de l'art pour présenter des propriétés macroscopiques qui posent des difficultés dans les opérations de manutention et transport du solide (mauvaise coulabilité du solide, mottage, faible résistance à un effort de cisaillement), de processing (mauvaise filtrabilité, difficulté de séchage, attrition).

Dans le cadre de l'acide 2-acrylamido-2-méthylpropane sulfonique, les problèmes supplémentaires qui sont rencontrés sont généralement la faible granulométrie des cristaux sous forme d'aiguilles, la densité du solide rencontrée, et le caractère explosif de la fine poussière.

Ces propriétés macroscopiques sont directement liées à la morphologie des cristaux et à leur surface spécifique. Dans le cas d'un cristal de forme aiguille, la surface spécifique est élevée.

Il est décrit dans les brevets WO 2009/072480, JP 2008/307822 et JP 2003/137857 que les cristaux de l'acide 2-acrylamido-2-méthylpropane sulfonique obtenus sont sous forme d'aiguilles.

### RESUME DE L'INVENTION

La présente invention a pour objet une forme spécifique de l'acide 2-acrylamido-2-méthylpropane sulfonique désignée ci-après « forme cristalline hydratée de l'acide 2-acrylamido-2-méthylpropane sulfonique ».

Un autre aspect de l'invention a pour objet le procédé de fabrication de la forme cristalline hydratée de l'acide 2-acrylamido-2-méthylpropane sulfonique.

Un autre aspect de l'invention a pour objet une solution aqueuse d'un sel de l'acide 2-acrylamido-2-méthylpropane sulfonique préparée à partir de la forme cristalline hydratée de l'acide 2-acrylamido-2-méthylpropane sulfonique.

Un autre aspect de l'invention a pour objet l'utilisation de la forme cristalline hydratée de l'acide 2-acrylamido-2-méthylpropane sulfonique pour la fabrication de (co)polymères hydrosolubles, hydrogonflants ou superabsorbants.

Un autre aspect de l'invention concerne l'utilisation de (co)polymères fabriqués à partir de la forme cristalline hydratée de l'acide 2-acrylamido-2-méthylpropane sulfonique et plus précisément dans le domaine de la récupération assistée du pétrole et du gaz.

**PRESENTATION DE L'INVENTION**

La présente invention a pour objet une forme cristalline hydratée de l'acide 2-acrylamido-2-méthylpropane sulfonique ayant un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta. L'incertitude de ces pics est généralement de l'ordre de 0.1°.

La cristallographie aux rayons X, radiocristallographie ou diffractométrie de rayons X est une technique d'analyse qui permet d'étudier la structure de la matière cristalline à l'échelle atomique. Elle s'appuie sur le phénomène physique de diffraction des rayons X. Un diffractomètre ayant une source au cuivre peut être utilisé.

Une poudre formée d'une phase cristalline donnée va toujours donner lieu à des pics de diffraction dans les mêmes directions. Ce diagramme de diffraction forme ainsi une véritable signature de la phase cristalline. Il est donc possible de déterminer la nature de chaque phase cristalline au sein d'un mélange ou d'un produit pur.

Cette signature est spécifique à chaque composé organique ou inorganique, et se présente sous la forme d'une liste de pics de position en angle $2\theta$ (2-thêta).

Cette technique est utilisée pour caractériser la matière, en particulier les différentes formes cristallines qui peuvent exister pour une même molécule chimique.

Un autre aspect de l'invention a pour objet une forme cristalline hydratée de l'acide 2-acrylamido-2-méthylpropane sulfonique ayant un spectre infra-rouge à transformée de Fourier comprenant des pics à $3280cm^{-1}$, $3126cm^{-1}$, $1657cm^{-1}$, $1595cm^{-1}$, $1453cm^{-1}$, $1395cm^{-1}$, $1307cm^{-1}$, $1205cm^{-1}$, $1164cm^{-1}$, $1113cm^{-1}$, $1041cm^{-1}$, $968cm^{-1}$, $885cm^{-1}$, $815cm^{-1}$, $794cm^{-1}$. L'incertitude de ces pics est généralement de l'ordre de $8cm^{-1}$. De manière avantageuse, il s'agit du spectre solide obtenu conventionnellement dans un sel tel que le KBr.

La spectroscopie infra-rouge à transformée de Fourier est l'analyse des vibrations émises, absorbées ou diffusées par les molécules. Cette technique est sensible aux interactions dites courtes (influence de la maille unitaire sur les liaisons). Dans la majorité des cas, les spectres infra-rouges à transformée de Fourier de différents systèmes cristallins diffèrent de manière significative. Le spectre infra-rouge à transformée de Fourier reflète donc les détails de la structure cristalline d'un composé organique.

De manière générale, et sauf indication contraire, le diagramme de diffraction des rayons X et le spectre infra-rouge sont obtenus à 20°C et à une pression de 1 atmosphère (101 325 Pa).

Un autre aspect de l'invention a pour objet une forme cristalline hydratée de l'acide 2-acrylamido-2-méthylpropane sulfonique ayant une énergie minimale d'inflammation supérieure à 400 mJ, préférentiellement supérieure à 500 mJ.

L'énergie minimale d'inflammation représente l'énergie minimale qui doit être fourni à un composé pour provoquer une inflammation. L'énergie peut être électrique ou thermique. L'énergie minimale d'inflammation est une donnée essentielle pour la prise en compte du risque d'explosion lors de la manipulation du produit (transfert, stockage, réaction, mise en forme...).

L'énergie minimale d'inflammation dépend des propriétés de la poudre (composition) ainsi que de sa structure macromoléculaire (granulométrie, forme cristalline, surface spécifique).

Dans le cadre des solides, cette énergie est l'énergie minimale d'une étincelle électrique susceptible d'enflammer un nuage de poussière. Plus la valeur de l'énergie minimale d'inflammation est élevée, moins le solide présente un risque lors de son utilisation, manipulation, stockage.

La mesure de l'énergie minimale d'inflammation est réalisée selon la norme NF EN 13821.

Un autre aspect de la présente invention a pour objet une forme cristalline hydratée de l'acide 2-acrylamido-2-méthylpropane sulfonique présentant 4 phénomènes thermiques avec la technique de calorimétrie différentielle à balayage, à 70°C, 100°C, 150°C et 190°C. L'incertitude relative à l'observation de ces phénomènes est généralement de l'ordre de 10°C, avantageusement 5°C ou moins.

Les phénomènes thermiques sont mesurés par calorimétrie différentielle à balayage (DSC). Cette technique exploite la mesure de la variation de chaleur associée à la dénaturation thermique du composé lorsque celui-ci est chauffé à vitesse constante, par exemple avec une rampe de chauffe de 10°C/minute.

Il est généralement reconnu que le phénomène thermique se présentant à 190°C (+/-10°C) est lié au point de fusion de l'acide 2-acrylamido-2-méthylpropane sulfonique.

De manière avantageuse, la forme cristalline hydratée de l'acide 2-acrylamido-2-méthylpropane sulfonique

selon l'invention présente un rapport molaire eau/acide 2-acrylamido-2-méthylpropane sulfonique égal à 1.

Un autre aspect de l'invention a pour objet le procédé de fabrication de la forme cristalline hydratée de l'acide 2-acrylamido-2-méthylpropane sulfonique comprenant au moins les étapes successives suivantes :

1) mélange d'acide 2-acrylamido-2-méthylpropane sulfonique avec une solution aqueuse, avantageusement pendant au moins 1 minute, afin de former une suspension A la solution aqueuse comprenant un solvant 1 organique et/ou un acide inorganique différent de l'acide 2-acrylamido-2-méthylpropane sulfonique,
2) chauffage de la suspension A, avantageusement à une température comprise entre 40 et 150°C, afin d'obtenir une solution B d'acide 2-acrylamido-2-méthylpropane sulfonique,
3) refroidissement de la solution B, avantageusement à une température comprise entre -40 et 100°C, avantageusement pendant une durée comprise entre 1 et 1200 minutes, afin d'obtenir une suspension C de cristaux,
4) séparation solide / liquide de la suspension C et isolement des cristaux de la suspension C obtenue à l'issue de l'étape 3) sous la forme d'une composition 1 dans laquelle les cristaux représentent avantageusement entre 40 et 99 % en poids de la composition 1. Les cristaux obtenus sont sous forme cristalline hydratée.

La température des étapes 2) et 3) peut varier en fonction notamment de la concentration en acide 2-acrylamido-2-méthylpropane sulfonique. L'homme du métier saura adapter la variation de température pour optimiser la formation des cristaux.

*Etape 1) :*

L'acide 2-acrylamido-2-méthylpropane sulfonique est issu d'un procédé de fabrication tel que décrit précédemment (acrylonitrile, acide sulfurique fumant et isobutylène). L'acide 2-acrylamido-2-méthylpropane sulfonique peut être sous forme de poudre fine ou mis en forme de manière contrôlée par un procédé tel que la compaction, ou la granulation, ou l'extrusion.

Le ratio en poids de solution aqueuse mélangée avec l'acide 2-acrylamido-2-méthylpropane sulfonique est avantageusement compris entre 0,1:1 et 5:1, plus préférentiellement entre 0,2:1 et 3:1.

Selon un mode de réalisation particulier, la solution aqueuse peut comprendre jusqu'à 20% en poids en solvant 1 organique, préférentiellement de 1 à 15 % en poids de solvant 1 organique, plus préférentiellement de 2 à 10% en poids de solvant 1 organique.

Selon un mode particulier de l'invention, la solution aqueuse comprend au moins 80% en poids d'eau et jusqu'à 20% en poids de solvant 1 organique, préférentiellement entre 85% et 99% en poids d'eau et de 1% à 15% en poids de solvant 1 organique, plus préférentiellement entre 90% et 98% en poids d'eau et 2% à 10% en poids de solvant 1 organique.

Le solvant 1 organique est choisi parmi les composés suivants :

- acides organiques, avantageusement les acides carboxyliques comprenant de 1 à 8 carbones,
- les amides comprenant avantageusement de 1 à 8 atomes de carbone,
- les alcools comprenant avantageusement de 1 à 8 atomes de carbones,
- les cétones comprenant avantageusement de 1 à 8 atomes de carbones,
- les éthers comprenant avantageusement de 1 à 8 atomes de carbones,
- les esters comprenant avantageusement de 1 à 8 atomes de carbones,
- les alcanes comprenant avantageusement de 1 à 8 atomes de carbones,
- les composés hydrocarbonés halogénés comprenant avantageusement de 1 à 8 atomes de carbones,
- les nitriles comprenant avantageusement de 1 à 8 atomes de carbones, ou
- leurs mélanges.

Ces composés peuvent être linéaires ou ramifiés. Ils peuvent être saturés ou comprendre des insaturations, par exemple C≡N dans le cas des nitriles.

Préférentiellement, le solvant 1 est choisi parmi l'acrylonitrile, l'isopropanol, l'acide acétique ou leurs mélanges. Préférentiellement, le solvant 1 est l'acrylonitrile.

Le solvant 1 est sous forme de liquide à la température de réalisation des étapes 2) et 3). En outre, il est avantageusement miscible dans l'eau.

Le solvant 1 peut, le cas échéant, permettre de solubiliser d'éventuelles impuretés ou sous-produits présents avec l'acide 2-acrylamido-2-méthylpropane sulfonique utilisé pour former la suspension A. En revanche, l'acide 2-acrylamido-2-méthylpropane sulfonique n'est pas nécessairement soluble dans le solvant 1.

Selon un autre mode particulier de l'invention, la solution aqueuse peut comprendre au moins 80% en poids d'eau et jusqu'à 20% en poids d'acide inorganique, préférentiellement entre 80% et 99% en poids d'eau et de 1% à 20

% en poids d'acide inorganique, plus préférentiellement entre 85% et 98% en poids d'eau et 2% à 15% en poids d'acide inorganique.

Préférentiellement, l'acide inorganique est l'acide sulfurique. Dans ce cas, la solution aqueuse d'acide sulfurique peut être préparée par dilution d'un acide contenant moins de 80% d'eau, ou d'une source de $SO_3$ tel que l'oléum ou le trioxyde de soufre.

Ainsi, la solution aqueuse comprend au moins un solvant organique 1 et/ou au moins un acide inorganique différent de l'acide 2-acrylamido-2-méthylpropane sulfonique.

Le temps de mélange entre la solution aqueuse et l'acide 2-acrylamido-2-méthylpropane sulfonique est avantageusement d'au moins 1 minute. Lors du mélange, l'ajout de la solution aqueuse peut se faire de manière séquentielle avant ou après l'acide 2-acrylamido-2-méthylpropane sulfonique. Lors du mélange, l'ajout de la solution aqueuse et de l'acide 2-acrylamido-2-méthylpropane sulfonique peut se faire de manière simultanée.

La température de mélange est généralement inférieure à 40°C. La limite basse de température est limitée par la température de fusion de la solution aqueuse ou de la suspension A.

Le mélange des produits de l'étape 1) peut s'effectuer par diverses technologies. A titre d'exemples et de manière non limitative, nous pouvons citer les réacteurs avec agitateur, les réacteurs boucle, les mélangeurs statiques, les microréacteurs, les réacteurs piston, les réacteurs filtres sécheurs agités, par exemple Nutsche, les mélangeurs à palettes, les mélangeurs bicônes, les mélangeurs à soc de charrue, et les mélangeurs à disques.

*Etape 2) :*

La suspension A obtenue à l'étape 1) est chauffée à une température comprise entre 40 et 150°C, plus préférentiellement entre 50 et 120°C afin d'obtenir une solution B.

Le temps de mise en température de la solution B n'a pas d'influence sur les bénéfices de l'invention.

La montée en température de la suspension A pour obtenir une solution B peut s'effectuer par diverses technologies. A titre d'exemples et de manière non limitative nous pouvons citer les réacteurs avec agitateur, les réacteurs boucle, les mélangeurs statiques, les microréacteurs, les réacteurs piston, les réacteurs filtres sécheurs agités, par exemple Nutsche, les échangeurs de chaleur, les mélangeurs à palettes, les mélangeurs bicônes, les mélangeurs à soc de charrue, les mélangeurs à disques, les évaporateurs à film tombant, les évaporateurs à film raclée, et les rebouilleurs.

L'étape 2) permet de solubiliser l'acide 2-acrylamido-2-méthylpropane sulfonique.

Dans un mode particulier de l'invention, la solution B peut subir une opération de séparation solide/liquide afin d'enlever toutes particules insolubles.

*Etape 3 :*

La solution B obtenue à l'étape 2) est refroidie à une température comprise entre -40 et 100°C, plus préférentiellement entre -20 et 50°C. Comme déjà indiqué, l'homme du métier saura ajuster la température en fonction de la concentration en acide 2-acrylamido-2-méthylpropane sulfonique et/ou en fonction du point de fusion du solvant 1 et/ou de l'acide inorganique de l'étape 1).

De manière générale, la température de l'étape 3) est inférieure à la température de l'étape 2).

Le temps de refroidissement est avantageusement compris entre 1 minute et 1200 minutes.

La vitesse de refroidissement peut ne pas être constante tout le long du processus. A titre d'exemple, la solution B peut être refroidie de 5°C par heure pendant les trois premières heures, et ensuite être refroidie à une vitesse de 10°C par heure jusqu'à l'obtention de la température finale.

Lors du refroidissement de la solution B, des cristaux d'acide 2-acrylamido-2-méthylpropane sulfonique sont formés et précipitent, pour former une suspension C.

Le refroidissement de la solution B pour obtenir une suspension C peut s'effectuer par diverses technologies. A titre d'exemples et de manière non limitative nous pouvons citer les réacteurs avec agitateur, les réacteurs boucle, les mélangeurs statiques, les microréacteurs, les réacteurs piston, les réacteurs filtres sécheurs agités, par exemple Nutsche, les échangeurs de chaleur, les mélangeurs à palettes, les mélangeurs bicônes, les mélangeurs à soc de charrue, les mélangeurs à disques, les évaporateurs à film tombant, les évaporateurs à film raclée, et les réacteurs non agités.

Dans un mode particulier de l'invention, des cristaux de forme hydratée de l'acide 2-acrylamido-2-méthylpropanesulfonique préalablement préparés peuvent être ajoutés lors de cette étape afin de modifier la formation de la suspension C. Il s'agit d'un ensemencement en cristallisation qui permet de mieux contrôler la température de cristallisation, la granulométrie des cristaux, la distribution granulométrique, la pureté du produit final et, éventuellement, le rendement.

*Etape 4 :*

Les cristaux d'acide 2-acrylamido-2-méthylpropane sulfonique hydraté contenus dans la suspension C obtenus à l'issue de l'étape 3) sont isolés par une étape de séparation liquide/solide et se présentent sous la forme d'une composition 1. A titre d'exemples et de manière non limitative, nous pouvons citer l'utilisation d'une centrifugeuse, d'un décanteur, d'un filtre presse, d'un filtre lisseur agité, d'un filtre à bande, d'un filtre à disques, ou d'un filtre à tambour rotatif. D'une manière préférentielle la séparation liquide/solide s'effectue à l'aide d'une centrifugeuse. La séparation liquide/solide peut également s'effectuer par décantation gravitaire.

L'étape 4) est avantageusement réalisée à une température comprise entre -40 et 100°C, plus préférentiellement entre -20 et 50°C.

Préférentiellement après l'étape 4) de séparation liquide/solide, les cristaux d'acide 2-acrylamido-2-méthylpropane sulfonique ne sont pas séchés.

La composition 1 isolée a un taux de cristaux d'acide 2-acrylamido-2-méthylpropane sulfonique avantageusement compris entre 40 et 99% plus préférentiellement entre 60 et 99% en poids, et encore plus préférentiellement entre 60 et 98% en poids. Le restant de la composition 1 comprend principalement de l'eau.

A l'issue de cette étape 4), les cristaux de l'acide 2-acrylamido-2-méthylpropane sulfonique sont caractérisés comme étant des cristaux sous forme hydratée de l'acide 2-acrylamido-2-méthylpropane sulfonique.

D'autre part, la phase liquide obtenue à la suite de la séparation liquide/solide contient principalement de l'eau et de l'acide 2-acrylamido-2-méthylpropane sulfonique à saturation, et minoritairement du solvant 1 organique et/ou de l'acide inorganique. Selon un mode particulier de l'invention, cette phase liquide après séparation peut être utilisée totalement ou partiellement dans la solution aqueuse en étape 1).

*Etape 5) :*

Dans une étape 5) optionnelle, la composition 1 contenant les cristaux obtenus à l'issue de l'étape 4) est lavée à l'aide d'une solution de lavage.

La solution de lavage est avantageusement une solution aqueuse qui peut comprendre jusqu'à 20% de solvant 1 organique.

Préférentiellement, la solution de lavage comprend au moins 80% en poids d'eau et jusqu'à 20% en poids de solvant 1 organique, préférentiellement entre 80% et 99% en poids d'eau et de 1% à 20 % en poids de solvant 1 organique, plus préférentiellement entre 85% et 98% en poids d'eau et 2% à 15% en poids de solvant 1 organique.

Comme déjà dit, le solvant 1 organique est choisi parmi les acides organiques, les amides, les alcools, les cétones, les éthers, les esters, les alcanes, les composés hydrocarbonés halogénés, les nitriles, ou leurs mélanges. Préférentiellement, le solvant 1 est choisi parmi l'acrylonitrile, l'isopropanol, l'acide acétique ou leurs mélanges. Plus préférentiellement, le solvant 1 est l'acrylonitrile.

Selon un mode particulier de l'invention, le lavage de la composition 1 obtenue à l'issue de l'étape 4) est effectué en pulvérisant de la solution de lavage sur ladite composition 1.

Selon un autre mode particulier de l'invention, le lavage de la composition 1 obtenue à l'issue de l'étape 4) est effectué en mettant en suspension la composition 1 dans la solution de lavage.

Le ratio en poids entre la solution aqueuse de lavage et la composition 1 obtenue à l'issue de l'étape 4) est avantageusement compris entre 0,05:1 et 10:1 et plus préférentiellement entre 0,1:1 et 5:1.

Cette étape de lavage est avantageusement réalisée à une température comprise entre -20 et 100°C. L'homme du métier saura ajuster la température de manière à ne pas solubiliser les cristaux sous forme hydratée d'acide 2-acrylamido-2-méthylpropane sulfonique.

Selon un mode particulier, la solution aqueuse de lavage peut comprendre jusqu'à 60% en poids d'acide 2-acrylamido-2-méthylpropane sulfonique.

Les cristaux sous forme hydratée d'acide 2-acrylamido-2-méthylpropane sulfonique obtenus à l'issue de cette étape 5) optionnelle peuvent être isolés de la solution de lavage par une étape de séparation liquide/solide, sous la forme d'une composition 2. A titre d'exemples et de manière non limitative, nous pouvons citer l'utilisation d'une centrifugeuse verticale ou horizontale, d'un décanteur, d'un filtre presse, d'un filtre à bande, d'un filtre à disques, d'un filtre poussoir, ou d'un filtre à tambour rotatif. La séparation liquide/solide peut également s'effectuer par décantation gravitaire.

Selon un mode particulier de l'invention, la solution de lavage récupérée peut être utilisée totalement ou partiellement de nouveau en étape 5), avec ou sans étape de traitement préalable.

Selon un mode particulier de l'invention, la solution de lavage récupérée peut être utilisée totalement ou partiellement dans la solution aqueuse en étape 1), avec ou sans étape de traitement préalable.

*Etape 6) :*

Dans une étape 6) optionnelle, la composition 2 obtenue à l'issue de l'étape 5) est séchée. A titre d'exemples et de manière non limitative, nous pouvons citer l'utilisation de toutes technologies de séchage par convection, conduction ou rayonnement (sécheur à lit fluidisé, lit traversé, séchage sur bande convoyeuse, séchage micro-onde séchage sur filtre lisseur agité chauffé, séchage par rayonnement haute fréquence, infra-rouge, séchage par atomisation).

L'opération de séchage peut être réalisée à pression atmosphérique ou bien sous vide.

L'étape de séchage peut être réalisée de manière batch ou en continu.

Pendant le procédé de fabrication, c'est-à-dire pendant les étapes 1) à 6), et quelle que soit l'étape, il est possible d'introduire au moins un inhibiteur de polymérisation de manière à éviter l'éventuelle polymérisation de l'acide 2-acrylamido-2-méthylpropane sulfonique. Cet inhibiteur peut être choisi de manière non limitative parmi l'hydroquinone, le paraméthoxyphénol, le phénotiazine, le 2,2,6,6-tétraméthyl(pipéridin-1-yl)oxyl, le 4-hydroxy-2,2,6,6-tétraméthylpipéridin-1-oxyl, les dérivés de phénylène diamines, ou leurs mélanges.

Préférentiellement, l'inhibiteur est le paraméthoxyphénol ou le 4-hydroxy-2,2,6,6-tétraméthylpipéridin-1-oxyl.

La quantité d'inhibiteur introduite par rapport à la quantité d'acide 2-acrylamido-2-méthylpropane sulfonique introduite à l'étape 1) est avantageusement comprise entre 0,001% et 5% en poids, plus préférentiellement entre 0,01% et 1% en poids.

L'inhibiteur peut être introduit au cours d'une ou plusieurs quelconques étapes du procédé. Préférentiellement, il est introduit en quantité supplémentaire pendant l'étape 1). Plus préférentiellement, l'inhibiteur fait partie de la solution aqueuse introduite en étape 1).

Le procédé de fabrication (étapes 1) à 6)) peut être effectué de manière continue ou discontinue (en batch).

Un autre aspect de l'invention a pour objet un procédé de préparation d'une solution aqueuse A d'un sel de l'acide 2-acrylamido-2-méthylpropane sulfonique préparé à partir de la forme cristalline hydratée.

Le procédé de préparation d'une solution aqueuse A de sel de l'acide 2-acrylamido-2-méthylpropane sulfonique comprend les étapes suivantes :

a) préparation d'une solution aqueuse X d'acide 2-acrylamido-2-méthylpropane sulfonique de concentration avantageusement comprise entre 1 et 700 g/L,

b) mise en contact et mélange de la solution aqueuse X avec un composé Y choisi parmi un hydroxyde de métal alcalin ou alcalino-terreux, un oxyde de métal alcalin ou alcalino-terreux, l'ammoniaque, une amine de formule suivante $NR_1R_2R_3$ ($R_1$, $R_2$ et $R_3$ étant avantageusement des groupements hydrocarbonés, notamment des alkyles) ou un carbonate de métal alcalin ou alcalino-terreux.

Etape a) :

La forme cristalline hydratée de l'acide 2-acrylamido-2-méthylpropane sulfonique peut être sous forme de poudre fine ou mise en forme de manière contrôlée par un procédé tel que la compaction, ou la granulation, ou l'extrusion.

La solution aqueuse X d'acide 2-acrylamido-2-méthylpropanesulfonique est avantageusement préparée en mélangeant la forme cristalline hydratée de l'acide 2-acrylamido-2-méthylpropane sulfonique et une solution aqueuse Z.

Le temps de mélange entre la solution aqueuse Z et l'acide 2-acrylamido-2-méthylpropane sulfonique est avantageusement d'au moins 1 minute. Lors du mélange, l'ajout de la solution aqueuse Z et de l'acide 2-acrylamido-2-méthylpropane sulfonique peut se faire de manière séquentielle, sans ordre de préférence, ou de manière simultanée.

La température de mélange est généralement inférieure à 60°C. La limite basse de température est limitée par la température de cristallisation de la solution aqueuse Z ou de la solution aqueuse X d'acide 2-acrylamido-2-méthylpropane sulfonique.

La solution aqueuse Z est composée principalement d'eau, et peut contenir de l'acide 2-acrylamido-2-méthylpropane sulfonique ou son sel préparé préalablement à partir d'une quelconque des bases préalablement listées (composé Y).

Le mélange des produits de l'étape a) peut s'effectuer par diverses technologies. A titre d'exemples et de manière non limitative, nous pouvons citer les réacteurs avec agitateur, les réacteurs boucle, les mélangeurs statiques, les microréacteurs, les réacteurs piston, les réacteurs filtres sécheurs agités, par exemple Nutsche, les mélangeurs à palettes, les mélangeurs bicônes, les mélangeurs à soc de charrue, et les mélangeurs à disques.

Etape b) :

Le composé Y peut être sous une forme solide ou sous une forme liquide.

Selon un mode particulier de l'invention, le composé Y est sous forme solide, préférentiellement sous forme de poudre ou mis en forme par un procédé tel que la compaction, ou la granulation, ou l'extrusion.

Selon un autre mode particulier le composé Y est sous forme liquide, préférentiellement sous la forme d'une solution aqueuse Y.

Lorsque le composé Y est un hydroxyde de métal alcalin ou alcalino-terreux, il peut être choisi parmi l'hydroxyde de sodium, l'hydroxyde de potassium, l'hydroxyde de lithium, l'hydroxyde de magnésium et l'hydroxyde de calcium.

Lorsque le composé Y est un oxyde de métal alcalino-terreux, il peut être choisi parmi l'oxyde de calcium et l'oxyde de magnésium.

Lorsque le composé Y est une amine de formule $NR_1R_2R_3$ où Ri, $R_2$ et $R_3$ sont indépendamment un atome d'hydrogène ou une chaîne carbonée contenant de 1 à 22 carbones, avantageusement une chaîne linéaire, Ri, $R_2$ et $R_3$ n'étant pas simultanément un atome d'hydrogène. De manière générale, l'ammoniaque ($NH_3$) est préférée aux amines de formule $NR_1R_2R_3$.

De manière préférentielle, le composé Y est une solution aqueuse d'hydroxyde de métal alcalin ou alcalino-terreux. Préférentiellement, l'hydroxyde de métal alcalin est l'hydroxyde de sodium.

Lorsque le composé Y est sous forme de solution aqueuse Y, la concentration de la solution en composé Y est avantageusement comprise entre 0, 1 et 70% massique.

Durant le mélange de la solution aqueuse X avec la solution aqueuse Y, la température est avantageusement maintenue entre -10 et 60 °C, préférentiellement entre 0 et 30 °C.

Le ratio molaire entre l'acide 2-acrylamido-2-méthylpropane sulfonique et le composé Y est avantageusement compris entre 1:0,1 et 1:1,1, plus préférentiellement entre 1:0,5 et 1:1,05.

Lors du mélange, l'ajout de la solution aqueuse X peut se faire de manière séquentielle avant ou après le composé Y (ou sa solution aqueuse Y). Lors du mélange, l'ajout de la solution aqueuse X et du composé Y (ou sa solution aqueuse) peut se faire de manière simultanée.

Préférentiellement, la solution aqueuse X est ajoutée en première, suivi du composé Y (ou sa solution aqueuse Y).

Il est possible, quelle que soit l'étape, d'introduire au moins un inhibiteur de polymérisation pendant le procédé de préparation de la solution A du sel de l'acide 2-acrylamido-2-méthylpropane sulfonique préparé à partir de la forme cristalline hydratée solide. Cet inhibiteur peut être choisi de manière non limitative parmi l'hydroquinone, le paraméthoxyphénol, le phénotiazine, le 2,2,6,6-tétraméthyl(pipéridin-1-yl)oxyl, le 4-hydroxy-2,2,6,6-tétraméthylpipéridin-1-oxyl, les dérivés de phénylène diamines, ou leurs mélanges.

Préférentiellement, l'inhibiteur est le paraméthoxyphénol.

Un autre aspect de l'invention a pour objet l'utilisation de la nouvelle forme cristalline hydratée de l'acide 2-acrylamido-2-méthylpropane sulfonique pour la fabrication de (co)polymères. Cet aspect de l'invention couvre également l'utilisation de sels de la forme cristalline hydratée de l'acide 2-acrylamido-2-méthylpropane sulfonique pour la fabrication de (co)polymères.

De manière tout à fait inattendue, la Demanderesse a découvert que le (co)polymère selon l'invention présente des propriétés améliorées notamment en termes de filtrabilité et de stabilité chimique et thermique améliorée par rapport à des polymères préparés à partir d'ATBS conventionnel. Ces propriétés s'avèrent particulièrement utiles dans les techniques de récupération assistée du pétrole ou du gaz issus de réservoirs conventionnels, de schistes ou de sables bitumineux.

La présente invention concerne donc également un (co)polymère obtenu à partir au moins d'acide 2-acrylamido-2-méthylpropane sulfonique, sous sa forme acide et/ou salifiée, au moins une partie de l'acide 2-acrylamido-2-méthylpropane sulfonique étant sous forme cristalline hydratée et ayant un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta(+/- 0.1°).

Il peut s'agir d'un copolymère (obtenu à partir de plusieurs monomères distincts) ou d'un homopolymère.

Le monomère acide 2-acrylamido-2-méthylpropane sulfonique sous sa forme cristalline hydratée peut être sous la forme acide et/ou salifiée, le sel étant avantageusement obtenu à partir d'un composé choisi parmi un hydroxyde de métal alcalin ou alcalino-terreux, un oxyde de métal alcalin ou alcalino-terreux, l'ammoniaque, une amine de formule suivante $NR_1R_2R_3$ ou un carbonate de métal alcalin ou alcalino-terreux.

De manière générale, la forme salifiée de monomères anioniques peut être obtenue avant et/ou pendant et/ou après leur polymérisation. Elle est avantageusement obtenue avant la polymérisation, notamment dans le cas de la forme cristalline hydratée de l'acide 2-acrylamido-2-méthylpropane sulfonique.

Selon un mode particulier de l'invention, le polymère est un homopolymère de la forme cristalline hydratée de l'acide 2-acrylamido-2-méthylpropane sulfonique.

Selon un autre mode particulier de l'invention, le (co)polymère est obtenu au moins à partir d'acide 2-acrylamido-2-méthylpropane sulfonique dont 50% à 100% sont sous la forme cristalline hydratée, plus avantageusement 70 à 100%, et encore plus avantageusement 100%.

Le (co)polymère selon l'invention est avantageusement obtenu à partir de 1 à 100 mol% d'acide 2-acrylamido-2-méthylpropane sulfonique, préférentiellement entre 2 et 60 mol% d'acide 2-acrylamido-2-méthylpropane sulfonique,

encore plus préférentiellement entre 3 et 25 mol% d'acide 2-acrylamido-2-méthylpropane sulfonique ; dont 50% à 100 % de l'acide 2-acrylamido-2-méthylpropane sulfonique sont avantageusement sous la forme cristalline hydratée, plus avantageusement 70 à 100%, et encore plus avantageusement 100%.

De manière générale, l'homme du métier saura, le cas échéant, ajuster la quantité des éventuels monomères additionnels (anionique et/ou cationique et/ou zwitterionique) listés ci-après pour atteindre 100mol%.

Selon un autre mode particulier de l'invention, le (co)polymère peut être obtenu à partir de'acide 2-acrylamido-2-méthylpropane sulfonique dont 50% à 100% sont avantageusement sous la forme cristalline hydratée (plus avantageusement 70 à 100%, et encore plus avantageusement 100%) et à partir d'au moins un monomère non ionique et/ou au moins un monomère anionique et/ou au moins un monomère cationique et/ou un monomère zwittérionique.

Selon un autre mode particulier de l'invention, le polymère est un copolymère de la forme cristalline hydratée de l'acide 2-acrylamido-2-méthylpropane sulfonique et d'au moins un monomère non-ionique.

Le monomère non-ionique peut notamment être choisi dans le groupe comprenant les monomères vinyliques solubles dans l'eau, et particulièrement l'acrylamide ; le N-isopropylacrylamide ; le N,N-diméthylacrylamide ; la N-vinylformamide ; l'acryloyl morpholine ; le N,N-diéthyle acrylamide ; le N-tert-butyl acrylamide ; le N-tert-octylacrylamide ; la N-vinylpyrrolidone ; la N-vinyl caprolactame ; la N-vinyl-imidazole, l'hydroxyéthyl méthacrylamide, l'hydroxypropylacrylate, l'isoprenol et la diacétone acrylamide. De manière avantageuse, le monomère non-ionique est l'acrylamide.

Selon un mode de réalisation particulier, le copolymère est avantageusement obtenu à partir de 1 à 99 mol% de monomère(s) non-ionique(s), de préférence entre 40 et 95 mol% et plus préférentiellement entre 45 et 90 mol%, par rapport au nombre total de monomères. Dans ce cas, le copolymère est avantageusement obtenu à partir de 0,1 à 99 mol% d'acide 2-acrylamido-2-méthylpropane sulfonique dont 50% à 100% sont sous la forme cristalline hydratée, plus avantageusement 70 à 100%, et encore plus avantageusement 100%.

Le ou les monomères anioniques peuvent présenter des fonctions acryliques, vinyliques, maléiques, fumariques, malonique, itaconique, allyliques et contenir un groupe carboxylate, phosphonate, phosphate, sulfate, sulfonate, ou un autre groupe à charge anionique. Le monomère anionique peut être sous forme acide et/ou sous forme de sel de métal alcalino-terreux, de sel de métal alcalin ou de sel d'ammonium. Des exemples de monomères convenables comprennent l'acide acrylique ; l'acide méthacrylique ; l'acide itaconique ; l'acide crotonique ; l'acide maléique ; l'acide fumarique, l'acide acrylamido undécanoïque, l'acide 3- acrylamido 3-méthylbutanoïque, l'anhydride maléique ; les monomères de type acide fort présentant par exemple une fonction de type acide sulfonique ou acide phosphonique tels que l'acide vinylsulfonique, l'acide vinylphosphonique, l'acide allylsulfonique, l'acide méthallylsulfonique, l'acide 2-méthylidenepropane-1,3-disulfonique, le 2-sulfoéthylméthacrylate, le sulfopropylméthacrylate, le sulfopropylacrylate, l'acide allylphosphonique, l'acide styrène sulfonique, l'acide 2-acrylamido-2-méthylpropane disulfonique ; et les sels de ces monomères comme leurs sels de métaux alcalins, de métaux alcalino-terreux, ou d'ammonium. Dans cette liste, les monomères mentionnés de type acide fort présentant une fonction de type acide sulfonique n'incluent pas la forme cristalline hydratée de l'acide 2-acrylamido-2-méthylpropane sulfonique selon l'invention.

Selon un mode de réalisation particulier, le copolymère est avantageusement obtenu à partir de 1 à 99 mol% de monomère(s) anionique(s), de préférence entre 2 et 60 mol%, et encore plus préférentiellement entre 3 et 25 mol%, par rapport au nombre total de monomères. Ces pourcentages incluent le monomère de forme cristalline hydratée de l'acide 2-acrylamido-2-méthylpropane sulfonique selon l'invention.

Le ou les monomères cationiques pouvant être utilisés dans le cadre de l'invention peuvent être choisis, notamment parmi les monomères du type acrylamide, acrylique, vinylique, allylique ou maléique possédant une fonction phosphonium ou ammonium quaternaire. On peut citer, en particulier et de façon non limitative, l'acrylate de diméthylaminoéthyle (ADAME) quaternisé, le méthacrylate de diméthylaminoéthyle (MADAME) quaternisé, le chlorure de diméthyldiallylammonium (DADMAC), le chlorure d'acrylamido propyltriméthyl ammonium (APTAC), et le chlorure de méthacrylamido propyltriméthyl ammonium (MAPTAC). L'agent de quaternisation peut être choisi parmi les chlorures d'alkyle, les sulfates de dialkyle ou les halogénures d'alkyle. Préférentiellement, l'agent de quaternisation est choisi parmi le chlorure de méthyle ou le diéthyle sulfate.

Le monomère zwitterionique peut être de type acrylamide, acrylique, vinylique, allylique ou maléique possédant une fonction amine ou ammonium quaternaire et une fonction acide de type carboxylique, sulfonique ou phosphorique. On peut citer, en particulier et de façon non limitative les dérivés de l'acrylate de diméthylaminoéthyl, tel que le 2-((2-(acryloyloxy)éthyl) diméthylammonio) éthane-1-sulfonate, le 3-((2-(acryloyloxy)éthyl) diméthylammonio) propane-1-sulfonate, le 4-((2-(acryloyloxy)éthyl) diméthylammonio) butane-1-sulfonate, le [2-(acryloyloxy)éthyl] (diméthylammonio) acetate, les dérivés du méthacrylate de diméthylaminoéthyle tel que le 2-((2-(méthacryloyloxy) éthyl) diméthylammonio) éthane-1-sulfonate, le 3-((2-(méthacryloyloxy) éthyl) diméthylammonio) propane-1-sulfonate, le 4-((2-(méthacryloyloxy) éthyl) diméthylammonio) butane-1-sulfonate, le [2-(méthacryloyloxy)éthyl] (diméthylammonio) acétate, les dérivés du diméthylamino propylacrylamide tel que le 2-((3-acrylamidopropyl) diméthylammonio) éthane-1-sulfonate, le 3-((3-acrylamidopropyl) diméthylammonio) propane-1-sulfonate, le 4-((3-acrylamidopropyl) diméthylammonio) butane-1-sulfonate, le [3-(acryloyloxy) propyl] (diméthylammonio) acétate, les dérivés du diméthylamino propyl méthylacrylamide tel que le 2-((3-méthacrylamidopropyl) diméthylammonio) éthane-1-sulfonate, le 3-((3-méthacrylamidopropyl) diméthylammonio) propane-1-sulfonate, le 4-((3-méthacrylamidopropyl) diméthylammonio) butane-1-sulfonate et le [3-(méthacryloyloxy)propyl] (diméthylammonio) acétate.

Des monomères présentant un caractère hydrophobe peuvent également être utilisés dans l'invention. Ils sont de préférence choisis dans le groupe constitué par les esters de l'acide (méth)acrylique présentant une chaîne alkyle, arylalkyle, propoxylée, éthoxylée, ou éthoxylée et propoxylée ; les dérivés du (méth)acrylamide présentant une chaîne alkyle, arylalkyle propoxylée, éthoxylée, éthoxylée et propoxylée, ou dialkyle ; les alkyl aryl sulfonates.

Lorsqu'on utilise un monomère présentant un caractère hydrophobe, sa quantité se situe avantageusement dans la plage comprise entre 0,001 et 3 % en mole par rapport à la quantité totale de monomères.

Les monomères présentant une fonction fluorescente peuvent également être utilisés dans le cadre de l'invention. Un monomère présentant une fonction fluorescente peut être détecté par n'importe quelle méthode appropriée, par exemple par fluorométrie avec un fluorimètre à longueur d'onde fixe. Généralement, la détection du monomère présentant une fonction fluorescente se fait aux maxima d'excitation et d'émission, qui peuvent être déterminés à l'aide d'un fluorimètre à balayage.

Les monomères présentant une fonction fluorescente sont choisis, par exemple, parmi les monomères de type styrène sulfonate de sodium ou styrène sulfonique.

Selon l'invention, le (co)polymère utilisé peut avoir une structure linéaire, branché, réticulé, star (en forme d'étoile) ou comb (en forme de peigne). Ces structures peuvent être obtenues par sélection au choix de l'amorceur, de l'agent de transfert, de la technique de polymérisation telle que la polymérisation radicalaire contrôlée dite RAFT (transfert de chaîne réversible par addition-fragmentation, de l'anglais « reversible-addition fragmentation chain transfer »), NMP (polymérisation en présence de nitroxydes, de l'anglais « Nitroxide Mediated Polymerization ») ou ATRP (polymérisation radicalaire par transfert d'atomes, de l'anglais « Atom Transfert Radical Polymerization »), de l'incorporation de monomères structuraux, de la concentration.

De manière générale, le (co)polymère ne nécessite pas de développement de procédé de polymérisation particulier. En effet, il peut être obtenu selon toutes les techniques de polymérisation bien connues par l'homme de métier. Il peut notamment s'agir de polymérisation en solution ; polymérisation en gel ; polymérisation par précipitation ; polymérisation en émulsion (aqueuse ou inverse) ; polymérisation en suspension ; polymérisation par extrusion réactive ; ou de polymérisation micellaire.

Selon un mode particulier de l'invention, le (co)polymère peut être post hydrolysé. La post hydrolyse est la réaction du (co)polymère après polymérisation. Cette étape consiste en la réaction des groupes fonctionnels hydrolysables des monomères non ioniques, tel que les fonctions amide ou ester, avec une base. Durant cette étape de post-hydrolyse du (co)polymère, le nombre de fonction acide carboxylique augmente. En effet, la réaction entre la base et les fonctions amides ou esters présentent dans le (co)polymère produit des groupes carboxylates.

Le (co)polymère peut se présenter sous forme liquide, gel ou solide lorsque sa préparation inclut une étape de séchage tel que le « spray drying » (séchage par pulvérisation), le séchage sur tambour, le séchage par rayonnement électromagnétique (micro-ondes, haute fréquence) ou encore le séchage en lit fluidisé.

Selon l'invention, le (co)polymère peut être linéaire, structuré ou réticulé. Par (co)polymère structuré, on désigne un (co)polymère non linéaire qui possède des chaînes latérales de manière à obtenir, lorsque ce (co)polymère est mis en solution dans l'eau, un fort état d'enchevêtrement conduisant à des viscosités à bas gradient très importantes.

Le (co)polymère peut en outre être structuré ou réticulé :

- par au moins un agent de structure, pouvant être choisi dans le groupe comprenant des monomères à insaturation polyéthylénique (ayant au minimum deux fonctions insaturées), comme par exemple les fonctions vinyliques, allyliques, acryliques et époxy et l'on peut citer par exemple le méthylène bis acrylamide (MBA), la triallyamine, le chlorure de tétraallylammonium, 1,2 dihydroxyéthylène bis-(N-acrylamide), et/ou
- par des macroamorceurs tels que les polyperoxydes, polyazoïques et les polyagents de transfert tels que les (co)polymères polymercaptants, les polyols, et/ou
- par des polysaccharides fonctionnalisés.

La quantité d'agent de ramification/réticulation dans le mélange de monomères est avantageusement inférieure à 4 % en poids par rapport à la teneur en monomères, plus avantageusement inférieure à 1 %, et encore plus avantageusement inférieure à 0,5 %. Selon un mode de réalisation particulier, elle peut être au moins égale à 0,00001 % en poids par rapport à la teneur en monomères.

Selon un mode de réalisation particulier, le (co)polymère peut comprendre au moins un groupe à LCST.

Selon les connaissances générales de l'homme du métier, un groupe à LCST correspond à un groupe dont la solubilité dans l'eau pour une concentration déterminée, est modifiée au-delà d'une certaine température et en fonction de la salinité. Il s'agit d'un groupe présentant une température de transition par chauffage définissant son manque d'affinité avec le milieu solvant. Le manque d'affinité avec le solvant se traduit par une opacification ou une perte de transparence qui peut être due à une précipitation, une agrégation, une gélification ou une viscosification du milieu. La

température de transition minimale est appelée « LCST » (température critique inférieure de solubilité, de l'anglais « Lower Critical Solution Température »). Pour chaque concentration de groupe à LCST, une température de transition par chauffage est observée. Elle est supérieure à la LCST qui est le point minimum de la courbe. En dessous de cette température, le (co)polymère est soluble dans l'eau, au-dessus de cette température, le (co)polymère perd sa solubilité dans l'eau.

Selon un mode de réalisation particulier, le (co)polymère peut comprendre au moins un groupe à UCST.

Selon les connaissances générales de l'homme du métier, un groupe à UCST correspond à un groupe dont la solubilité dans l'eau pour une concentration déterminée, est modifiée en dessous d'une certaine température et en fonction de la salinité. Il s'agit d'un groupe présentant une température de transition par refroidissement définissant son manque d'affinité avec le milieu solvant. Le manque d'affinité avec le solvant se traduit par une opacification ou une perte de transparence qui peut être due à une précipitation, une agrégation, une gélification ou une viscosification du milieu. La température de transition maximale est appelée « UCST » (température critique supérieure de solubilité, de l'anglais « Upper Critical Solution Température »). Pour chaque concentration de groupe à UCST, une température de transition par refroidissement est observée. Elle est inférieure à la UCST qui est le point maximum de la courbe. Au-dessus de cette température, le (co)polymère est soluble dans l'eau, en-dessous de cette température, le (co)polymère perd sa solubilité dans l'eau.

Selon l'invention, le (co)polymère a un poids moléculaire avantageusement élevé. Par « poids moléculaire élevé », on désigne des poids moléculaires d'au moins 1 million de g/mol, préférentiellement entre 2 et 40 millions g/mol, plus préférentiellement entre 5 et 30 millions g/mol. Le poids moléculaire s'entend en poids moléculaire moyen en poids.

Selon un mode de réalisation de l'invention, le (co)polymère peut être obtenu par (co)polymérisation d'au moins un monomère hydrosoluble et d'au moins un monomère de forme cristalline hydratée de l'acide 2-acrylamido-2-méthylpropane sulfonique et/ou d'un de ses sels.

De manière générale, et sauf contre-indication, par « acide 2-acrylamido-2-méthylpropane sulfonique sous forme cristalline hydratée » on désigne la forme acide et/ou la forme salifiée. Il en est de même pour les monomères anioniques mentionnés dans la description de l'invention qui peuvent désigner les formes acides et/ou salifiées comme, par exemple, pour l'acide acrylique.

La forme saline est avantageusement obtenue à partir d'un composé choisi parmi un hydroxyde de métal alcalin ou alcalino-terreux, un oxyde de métal alcalin ou alcalino-terreux, l'ammoniaque, une amine de formule suivante $NR_1R_2R_3$ (Ri, $R_2$ et $R_3$ étant avantageusement des groupements hydrocarbonés, notamment des alkyles) ou un carbonate de métal alcalin ou alcalino-terreux. Un métal alcalin préféré est le sodium.

La forme acide d'un monomère peut être salifiée avant et/ou pendant et/ou après la (co)polymérisation du ou des monomères.

Le (co)polymère selon l'invention est avantageusement hydrosoluble.

De manière avantageuse, le (co)polymère a un poids moléculaire compris entre 5000 et 40,000,000 g/mol, de préférence 1,250,000 et 35,000,000, et encore plus préférentiellement entre 2,750,000 et 30,000,000 g/mol en poids.

Le poids moléculaire est déterminé par la viscosité intrinsèque du (co)polymère. La viscosité intrinsèque peut être mesurée par des méthodes connues de l'homme du métier et peut être calculée à partir des valeurs de viscosité réduite pour différentes concentrations en (co)polymère par méthode graphique consistant à relever les valeurs de viscosité réduite (axe des ordonnées) sur la concentration (axe des abscisses) et d'extrapoler la courbe jusqu'à concentration nulle. La valeur de viscosité intrinsèque est relevée sur l'axe des ordonnées ou en utilisant la méthode des moindres carrés. Le poids moléculaire peut alors être déterminé par l'équation de Mark-Houwink :

$$[\eta] = K\ M^{\alpha}$$

$[\eta]$ représente la viscosité intrinsèque du (co)polymère déterminée par la méthode de mesure de viscosité en solution.
K représente une constante empirique.
M représente le poids moléculaire du (co)polymère.
$\alpha$ représente le coefficient de Mark-Houwink.
K et $\alpha$ dépendent du système particulier (co)polymère-solvant.

Un autre aspect de l'invention concerne l'utilisation de (co)polymères fabriqués à partir de la forme cristalline hydratée de l'acide 2-acrylamido-2-méthylpropane sulfonique ou d'au moins un de ses sels. Dans ces (co)polymères, l'acide 2-acrylamido-2-méthylpropane sulfonique peut être partiellement neutralisé, avant, ou pendant, ou après la (co)polymérisation de l'acide 2-acrylamido-2-méthylpropane sulfonique. Plus précisément, l'invention concerne l'utilisation de ces (co)polymères dans l'industrie du pétrole et du gaz, de la fracturation hydraulique, du papier, du traitement de l'eau, de la construction, de l'industrie minière, des cosmétiques, du textile ou de la détergence. Préférentiellement, les (co)polymères sont utilisés dans le domaine de la récupération assistée du pétrole et du gaz.

L'invention et les avantages qui en découlent ressortiront mieux des figures et exemples suivants donnés afin d'illustrer l'invention et non de manière limitative.

## DESCRIPTION DES FIGURES

La figure 1 illustre le spectre RMN du proton des cristaux d'acide 2-acrylamido-2-méthylpropane sulfonique obtenus selon les exemples 1 et 2.
La figure 2 illustre le diagramme par diffraction des rayons X des cristaux obtenus selon l'exemple 1.
La figure 3 illustre le diagramme par diffraction des rayons X des cristaux obtenus selon l'exemple 2.
La figure 4 illustre le spectre infra-rouge à transformée de Fourier des cristaux obtenus en exemple 1.
La figure 5 illustre le spectre infra-rouge à transformée de Fourier des cristaux obtenus selon l'exemple 2.
La figure 6 illustre le thermogramme des cristaux obtenus selon l'exemple 1.
La figure 7 illustre le thermogramme des cristaux obtenus selon l'exemple 2.
La figure 8 illustre le graphique de granulométrie des cristaux obtenus selon l'exemple 1.
La figure 9 illustre le graphique de granulométrie des cristaux obtenus selon l'exemple 2.
La figure 10 correspond à l'observation au microscope optique des cristaux obtenus selon l'exemple 1.
La figure 11 correspond à l'observation au microscope optique des cristaux obtenus selon l'exemple 2.
La figure 12 correspond à l'observation au microscope électronique à balayage des cristaux obtenus selon l'exemple 1.
La figure 13 correspond à l'observation au microscope électronique à balayage des cristaux obtenus selon l'exemple 2.
La figure 14 illustre la perte de viscosité en fonction de la forme de l'ATBS et de la teneur en fer pour un copolymère.
La figure 15 illustre la perte de viscosité en fonction de la forme de l'ATBS en vieillissement à 90°C pour un copolymère.
La figure 16 illustre la perte de viscosité en fonction de la forme de l'ATBS et de la teneur en fer pour un homopolymère.

## EXEMPLES DE REALISATION DE L'INVENTION

Exemple 1 : Synthèse de l'acide 2-acrylamido-2-méthylpropane sulfonique

Dans un réacteur agité de 2000 ml ayant une double enveloppe, sont ajoutés 1522 grammes d'acrylonitrile contenant 0,4% d'eau en poids et 180 grammes d'acide sulfurique fumant titrant 104% $H_2SO_4$ (18% Oléum). Le mélange est agité pendant 1 heure et refroidi par la double enveloppe du réacteur qui maintient la température du mélange sulfonant à -20°C. 97 grammes d'isobutylène sont additionnés au mélange sulfonant précédent, à un débit de 1,6 grammes/minute.

La température du mélange est contrôlée à 45°C lors de l'introduction de l'isobutylène. Les particules de l'acide 2-acrylamido-2-méthylpropane sulfonique précipitent dans le mélange et le taux de solide est d'environ 20% en poids. Le mélange réactionnel est filtré sur un filtre de type Büchner et séché sous vide à 50°C. Le solide obtenu est l'acide 2-acrylamido-2-méthylpropane sulfonique et se présente sous forme de poudre blanche très fine.

D'après les observations faites au microscope optique (figure 10) et au microscope électronique à balayage (figure 12), les cristaux ont une morphologie d'aiguille.

Exemple 2 : Formation de la forme cristalline hydratée de l'acide 2-acrylamido-2-méthylpropane sulfonique

Dans un réacteur agité de 2000 ml ayant une double enveloppe, sont ajoutés 500 grammes d'acide 2-acrylamido-2-méthylpropane sulfonique obtenu à l'exemple 1 et 460 grammes d'acide sulfurique à une concentration de 10% en $H_2SO_4$.

250 mg de 4-hydroxy-2,2,6,6-tétraméthylpipéridin-1-oxyl sont ajoutés au mélange précédant.

Le mélange est agité pendant 10 minutes, à 20°C, pour former une suspension A.

La suspension A est chauffée à une température de 60°C et est maintenue à cette température pendant 20 minutes afin de former une solution B.

La solution B est refroidie à une température de 10°C. Le temps de refroidissement entre 60°C et 10°C est de 6 heures. Une suspension C de cristaux d'acide 2-acrylamido-2-méthylpropane sulfonique est obtenue. La suspension C est filtrée sur une essoreuse verticale de marque Robatel. Un solide de composition 1 est obtenu, il contient 80% en poids de cristaux d'acide 2-acrylamido-2-méthylpropane sulfonique.

D'après les observations faites au microscope optique (figure 11) et au microscope électronique à balayage

(figure 13), les cristaux ont une morphologie de type cubique.

Exemple 3 : Analyse RMN des produits issus des exemples 1 et 2

Le solide de 2-acrylamido-2-méthylpropane sulfonique obtenu à l'exemple 1 et sa forme cristalline hydratée obtenue à l'exemple 2 sont analysés par résonance magnétique nucléaire (RMN) du proton.

Les échantillons sont dissous dans du $D_2O$. L'appareil de RMN a une fréquence de 400 MHZ, de marque Bruker, et est équipée d'une sonde 5mm BBO BB-$^1$H.

Les 2 spectres du proton (figure 1) sont identiques et l'assignement des pics est conforme à la structure moléculaire de l'acide 2-acrylamido-2-méthylpropane sulfonique.

Exemple 4 : Analyse par diffraction aux rayons X

Les solides obtenus aux exemples 1 et 2 sont préalablement broyés pour former des poudres et sont analysés par diffraction aux rayons X sur une gamme angulaire de 10 à 90°. L'équipement utilisé est un diffractomètre miniflex II de marque Rigaku et est équipé d'une source au cuivre.

Nous pouvons observer que le solide obtenu à l'issu de l'exemple 2 (figure 3) a un diagramme de diffraction aux rayons X ayant les pics caractéristiques suivants :
10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta (+/-0.1°).

Exemple 5 : Mesure infra-rouge à transformée de Fourier

L'équipement de mesure infra-rouge par transformée de Fourier est le Spectrum 100 de la marque Perkin Elmer, dont la précision est de 8cm$^{-1}$.

Les solides obtenus aux exemples 1 et 2 sont tamisés à 100$\mu$m. Les particules restant sur le tamis sont séchées et mises à l'étuve à 60°C pendant au moins 4 heures.

10 mg de solide sont précisément pesés et sont mélangés avec 500 mg de bromure de potassium (KBr). Le mélange est ensuite compacté dans une presse hydraulique sous une pression d'au moins 10 bars.

Nous pouvons observer que les bandes suivantes (figure 5) sont caractéristiques de la forme cristalline hydratée de l'acide 2-acrylamido-2-méthylpropane sulfonique : 3280cm$^{-1}$, 3126cm$^{-1}$, 1657cm$^{-1}$, 1595cm$^{-1}$, 1453cm$^{-1}$, 1395cm$^{-1}$, 1307cm$^{-1}$, 1205cm$^{-1}$, 1164cm$^{-1}$, 1113cm$^{-1}$, 1041cm$^{-1}$, 968cm$^{-1}$, 885cm$^{-1}$, 815cm$^{-1}$, 794cm$^{-1}$.

Le spectre infra-rouge du solide selon l'exemple 1 (figure 4) ne présente pas les mêmes pics.

Exemple 6 : Analyse différentielle calorimétrique (DSC)

L'équipement utilisé est un DSC131 EVO de la marque Setaram.

Les solides obtenus aux exemples 1 et 2 sont analysés avec une rampe de chauffe de 10°C/minute sous un flux d'azote. La température initiale est de 30°C, le produit est chauffé jusqu'à 220°C.

Le thermogramme des cristaux de l'exemple 1 (figure 6) montre un effet thermique à une température de 191,5°C, qui est généralement considéré comme le point de fusion/dégradation de l'acide 2-acrylamido-2-méthylpropane sulfonique.

Le thermogramme des cristaux de l'exemple 2 (figure 7) montrent 3 phénomènes thermiques additionnels visibles à 70,8 ; 103,4 et 152,2°C. Le pic à 187,4°C est lié à la dégradation de la molécule de l'acide 2-acrylamido-2-méthylpropane sulfonique.

En comparatif, le thermogramme des cristaux de l'exemple 1 ne présente qu'un pic de dégradation à 191,5°C (figure 6).

Exemple 7: Titration acido-basique

Dans un bécher de 1000ml sont introduits 500ml d'eau déminéralisée, ainsi que 100g du solide obtenu à l'exemple 1. Un barreau magnétique est ajouté afin de pouvoir mélanger la solution.

Une burette graduée est remplie avec de la soude 30%.

Un pH-mètre est ajouté afin de pouvoir suivre le pH lors de la coulée de la soude.

Initialement le pH de la solution aqueuse est inférieur à 1. De la soude est ajoutée jusqu'à obtention d'un pH=7.

64g de soude 30% sont ajoutés.

La masse molaire de l'acide 2-acrylamido-2-méthylpropane sulfonique est de 207g/mol. Le calcul du point d'équivalence montre que le solide obtenu à l'exemple 1 contient 99% en poids d'acide 2-acrylamido-2-méthylpropane

sulfonique (titration fonction acide).

Le solide obtenu à l'exemple 2 est titré selon le même protocole. 59g de soude sont ajoutés à 100g du solide obtenu à l'exemple 2. Le calcul du point d'équivalence montre que le solide obtenu à l'exemple 2 contient 92% en poids d'acide 2-acrylamido-2-méthylpropane sulfonique.

Les 8% restant sont de l'eau. Ce rapport massique acide 2-acrylamido-2-méthylpropane sulfonique/$H_2O$ (92/8) correspond à un rapport molaire 1:1.

Le solide obtenu dans l'exemple 2 est donc une forme cristalline hydratée de l'acide 2-acrylamido-2-méthylpropane sulfonique.

Exemple 8 : Mesure de l'énergie minimale d'inflammation (EMI)

La mesure de l'énergie minimale d'inflammation est réalisée selon la norme NF EN 13821.

L'explosimètre est un tube vertical de Hartmann. Le système de dispersion de poussières est de type champignon.

L'induction totale est inférieure à 25 micro Henry. La tension de décharge est comprise en 5kV et 15kV. Les électrodes sont en laiton et espacées d'une distance minimum de 6mm.

Différentes énergies et masse dispersées ont été testées et récapitulées dans les tableaux suivants.

Il apparait clairement que la forme cristalline hydratée présente un risque d'explosion bien moindre que la forme aiguille obtenue à l'exemple 1.

Tableau 1 : Détermination de l'EMI solide de l'exemple 1

| Energie (mJ) | Masse de solide dispersée (g) | Nombre de dispersion | Inflammation ? Oui (O) Non (N) | Flamme | Pression |
|---|---|---|---|---|---|
| 1000 | 0,5 | 2 | O | Petite | Petite |
| 500 | 0,5 | 3 | O | Moyenne | Moyenne |
| 300 | 0,5 | 3 | O | Moyenne | Moyenne |
| 100 | 0,5 | 20 | N | | |
| 200 | 0,5 | 20 | N | | |
| 200 | 1 | 20 | N | | |
| 200 | 2 | 20 | N | | |
| 200 | 3 | 7 | O | Moyenne | Petite |
| 100 | 3 | 20 | N | | |
| 100 | 5 | 20 | N | | |
| 100 | 7 | 20 | N | | |
| 100 | 10 | 20 | N | | |
| 100 | 1 | 20 | N | | |
| 100 | 2 | 20 | N | | |

Tableau 2 : Détermination de l'EMI solide de l'exemple 2

| Energie (mJ) | Masse de solide dispersée (g) | Nombre de dispersion | Inflammation ? Oui (O) Non (N) | Flamme | Pression |
|---|---|---|---|---|---|
| 1000 | 0,5 | 20 | N | | |
| 1000 | 1 | 20 | N | | |
| 1000 | 2 | 20 | N | | |
| 1000 | 3 | 20 | N | | |
| 1000 | 5 | 20 | N | | |

(suite)

| Energie (mJ) | Masse de solide dispersée (g) | Nombre de dispersion | Inflammation ? Oui (O) Non (N) | Flamme | Pression |
|---|---|---|---|---|---|
| 1000 | 7 | 20 | N | | |
| 1000 | 10 | 20 | N | | |
| 1000 | 15 | 13 | O | Petite | Moyenne |
| 500 | 15 | 20 | N | | |
| 500 | 20 | 20 | N | | |
| 500 | 10 | 20 | N | | |
| 500 | 7 | 20 | N | | |
| 500 | 5 | 20 | N | | |
| 500 | 3 | 20 | N | | |
| 500 | 2 | 20 | N | | |
| 500 | 1 | 20 | N | | |
| 500 | 0,5 | 20 | N | | |

Exemple 9 : mesure granulométrique

Les solides obtenus aux exemples 1 et 2 sont analysés par diffraction laser afin de déterminer leur répartition granulométrique.

L'équipement utilisé est un Cilas 1190.

Pour les cristaux de l'exemple 1, la valeur de $d_{50}$ est d'environ 40μm et 90% des particules ont une taille inférieure à 200μm (figure 8).

Pour les cristaux de l'exemple 2, la valeur de $d_{50}$ est d'environ 600μm et 90% des particules ont une taille inférieure à 1500μm (figure 9). Les cristaux contiennent moins de 10% de particules ayant une taille inférieure à 300μm.

Exemple 10 : Mesure de la surface spécifique

Les solides obtenus aux exemples 1 et 2 sont dégazés à température ambiante pendant 24 heures.

L'appareil de mesure de surface spécifique par sorptometrie est un appareil TriStar II Micromeritics couplé à un Smart VacPrep Micromeritics. La température de la mesure se fait à -196°C.

Tableau 3 : Surface spécifique des acides 2-acrylamido-2-méthylpropane sulfonique

| Origine de l'acide 2-acrylamido-2-méthylpropane sulfonique | Surface spécifique ($m^2/g$) |
|---|---|
| Exemple 1 (contre-exemple) | 1,32 +/-0,14 |
| Exemple 2 (invention) | 0,06 +/- 0,01 |

Exemple 11 : Protocole de préparation du sel de sodium de la forme cristalline hydratée de l'acide 2-acrylamido-2-méthylpropane sulfonique

Dans un réacteur de 2000ml ayant une double enveloppe, muni d'un condenseur, d'un pH-mètre et d'un agitateur, sont introduits 500 grammes de la forme cristalline hydratée de l'acide acrylamido-2-méthylpropane sulfonique de l'exemple 2 et 770 grammes d'eau. Le mélange a un pH inférieur à 1.

Dans une ampoule de coulée est préparée une solution d'hydroxyde de sodium de 50% en poids de concentration. La solution caustique est additionnée au mélange réactionnel, pendant 120 minutes. La température est contrôlée pour être inférieure à 30°C.

Pendant l'addition de l'hydroxyde de sodium le pH reste inférieur à 5.

175 grammes de solution d'hydroxyde de sodium de 50% en poids de concentration sont additionnés.

Le mélange obtenu est une solution de sel de sodium de l'acide 2-acrylamido-2-méthylpropane sulfonique à

une concentration de 35% en poids.

Exemple 12 : Préparation du copolymère P1 acrylamide/acide 2-acrylamido-2-méthylpropane sulfonique de forme cristalline hydratée (75/25 mole%)

Dans un bécher de 2000 ml sont ajoutés 549,5 g d'eau déionisée, 520,5 d'acrylamide en solution à 50%, 97,6 g de lessive de soude à 50%, 16,2 g d'urée et 316,2 g de cristaux d'acide 2-acrylamido-2-méthylpropane sulfonique obtenus dans l'exemple 2.

La solution ainsi obtenue est refroidie entre 0 et 5°C et transférée dans un réacteur de polymérisation adiabatique, un bullage d'azote est réalisé pendant 30 minutes afin d'éliminer toute trace d'oxygène dissout.

Sont ensuite ajoutés dans le réacteur :

- 0,75 g de 2,2'-azobisisobutyronitrile,
- 1.5 ml d'une solution à 5 g/l de de dihydrochlorure de 2,2'-azobis[2-(2-imidazolin-2-yl)propane],
- 1,5 ml d'une solution à 3 g/l d'hypophosphite de sodium,
- 2,25 ml d'une solution à 1 g/l de ter-butyl hydroperoxyde,
- 2,25 ml d'une solution à 1 g/l de sulfate d'ammonium et de fer(II) hexahydraté (sel de Mohr).

Après quelques minutes l'arrivée d'azote est fermée et le réacteur est clos. La réaction de polymérisation se déroule pendant 1 à 5 heures jusqu'à atteindre un pic de température. Le gel caoutchouteux obtenu est haché en particules d'une taille comprise entre 1 et 6 mm.

Le gel est ensuite séché et broyé pour obtenir le polymère sous forme de poudre.

Exemple 13 : Préparation du copolymère P'1 acrylamide/acide 2-acrylamido-2-méthylpropane sulfonique de forme non cristalline hydratée (75/25 mole%)

Le copolymère est préparé comme dans l'exemple 12 en remplaçant l'acide 2-acrylamido-2-méthylpropane sulfonique de forme cristalline hydratée (exemple 2) par de l'acide 2-acrylamido-2-méthylpropane sulfonique n'étant pas de forme cristalline hydratée obtenue en exemple 1.

Exemple 14 : Mesure de la résistance à la dégradation chimique de solutions de copolymères P1 et P'1 de poids moléculaire équivalents

Des tests de résistance à la dégradation chimique des polymères P1 et P'1 de poids moléculaire de 9 millions ont été réalisées en conditions aérobiques en présence de différentes concentrations en fer(II) (2, 5, 10 et 20 ppm) dans une saumure composée d'eau, de 37 000 ppm de NaCl, 5 000 ppm de $Na_2SO_4$ et 200 ppm de $NaHCO_3$. Ces tests ont été réalisés sur un polymère préparé à partir de la forme non cristalline de l'acide 2-acrylamido-2-méthylpropane sulfonique ou d'au moins un de ses sels (P'1) et sur un polymère préparé à partir de la forme cristalline de l'acide 2-acrylamido-2-méthylpropane sulfonique ou d'au moins un de ses sels (P1). Les deux polymères ont la même composition chimique. Les résultats obtenus après 24 h de mise en contact de la solution de polymère avec le contaminant sont présentés sur la figure 14.

Nous pouvons observer que, pour chaque concentration en fer(II), le polymère P1 perd moins de viscosité que le polymère P'1 équivalent.

Exemple 15 : Mesure de la résistance à la dégradation thermique de solutions de polymères de poids moléculaire équivalents

Des tests de résistance à la dégradation thermique des polymères P1 et P'1 de poids moléculaire de 9 millions ont été réalisés en conditions anaérobiques à une concentration active de 2 000 ppm dans une saumure composée d'eau, de 30 000 ppm de NaCl et 3 000 ppm de $CaCl_2,2H_2O$. Ces tests ont été réalisés sur un polymère fabriqué à partir de la forme non cristalline de l'acide 2-acrylamido-2-méthylpropane sulfonique ou d'au moins un de ses sels (P'1) et sur un polymère fabriqué à partir de la forme cristalline de l'acide 2-acrylamido-2-méthylpropane sulfonique ou d'au moins un de ses sels (P1). Les deux polymères ont la même composition chimique. Les solutions de polymères ont été vieillies pendant 6 mois à 90°C. Les résultats obtenus sont présentés sur la figure 15 en termes de perte de viscosité. Nous pouvons observer que le polymère P1 perd moins de viscosité que le polymère P'1 équivalent.

Exemple 16 : Préparation d'homopolymères P2 à partir de la forme cristalline hydratée de l'acide 2-acrylamido-2-méthylpropane sulfonique

Dans un bécher de 2000 ml sont ajoutés 390.5 g d'eau déionisé, 262 g de lessive de soude à 50% et 847.5 g de cristaux d'acide 2-acrylamido-2-méthylpropane sulfonique obtenu dans l'exemple 2.

La solution ainsi obtenue est refroidie entre 5 et 10°C et transférée dans un réacteur de polymérisation adiabatique, un bullage d'azote est réalisé pendant 30 minutes afin d'éliminer toute trace d'oxygène dissout.

Sont ensuite ajoutés dans le réacteur :

- 0,45 g de 2,2'-azobisisobutyronitrile,
- 1,5 ml d'une solution à 2.5 g/l de dihydrochlorure de 2,2'-azobis[2-(2-imidazolin-2-yl)propane],
- 1,5 ml d'une solution à 1 g/l d'hypophosphite de sodium,
- 1,5 ml d'une solution à 1 g/l de ter-butyl hydroperoxyde,
- 1,5 ml d'une solution à 1 g/l de sulfate d'ammonium et de fer(II) hexahydraté (sel de Mohr).

Après quelques minutes l'arrivée d'azote est fermée et le réacteur est clos. La réaction de polymérisation se déroule pendant 2 à 5 heures jusqu'à atteindre un pic de température. Le gel caoutchouteux obtenu est haché et séché pour obtenir une poudre grossière elle-même broyée et tamisée pour obtenir le polymère sous forme de poudre.

Exemple 17 : Préparation d'homopolymères P'2 à partir de l'acide 2-acrylamido-2-méthylpropane sulfonique de forme non cristalline hydratée

Les polymères sont préparés comme dans l'exemple 16 en remplaçant l'acide 2-acrylamido-2-méthylpropane sulfonique de forme cristalline hydratée par de l'acide 2-acrylamido-2-méthylpropane sulfonique n'étant pas de forme cristalline hydratée synthétisé en exemple 1.

Exemple 18 : Mesure de la résistance à la dégradation chimique de solutions des polymères P2 et P'2

Des tests de résistance à la dégradation chimique des polymères P2 et P'2 de poids moléculaire de 5,3 millions Da ont été réalisées en conditions aérobiques en présence de différentes concentrations en fer(II) (2, 5, 10 et 20 ppm) dans une saumure composée d'eau, de 37 000 ppm de NaCl, 5 000 ppm de $Na_2SO_4$ et 200 ppm de $NaHCO_3$. Ces tests ont été réalisés sur un polymère préparé à partir de la forme non cristalline de l'acide 2-acrylamido-2-méthylpropane sulfonique ou d'au moins un de ses sels (P'2) et sur un polymère préparé à partir de la forme cristalline de l'acide 2-acrylamido-2-méthylpropane sulfonique ou d'au moins un de ses sels (P2). Les deux polymères ont la même composition chimique. Les résultats obtenus après 24 h de mise en contact de la solution de polymère avec le contaminant sont présentés sur la figure 16.

Nous pouvons observer que, pour chaque concentration en fer(II), le polymère P2 perd moins de viscosité que le polymère P'2 équivalent.

Exemple 19 : Préparation du copolymère P3 acrylamide/acide 2-acrylamido-2-méthylpropane sulfonique de forme cristalline hydratée (75/25 mole%) post hydrolysé

Dans un bécher de 2000 ml sont ajoutés 761,9 g d'eau déionisé, 574,2g d'acrylamide en solution à 50%, 35,9g de lessive de soude à 50%, 11,7g d'urée et 116,3g de cristaux d'acide 2-acrylamido-2-méthylpropane sulfonique obtenus dans l'exemple 2.

La solution ainsi obtenue est refroidie entre 0 et 5°C et transférée dans un réacteur de polymérisation adiabatique, un bullage d'azote est réalisé pendant 30 minutes afin d'éliminer toute trace d'oxygène dissout.

Sont ensuite ajoutés dans le réacteur :

- 0,45 g de 2,2'-azobisisobutyronitrile,
- 1,5 ml d'une solution à 5 g/l de dihydrochlorure de 2,2'-azobis[2-(2-imidazolin-2-yl)propane],
- 1,5 ml d'une solution à 1 g/l d'hypophosphite de sodium,
- 2,25 ml d'une solution à 1 g/l de ter-butyl hydroperoxyde,
- 3,0 ml d'une solution à 1 g/l de sulfate d'ammonium et de fer(II) hexahydraté (sel de Mohr).

Après quelques minutes l'arrivée d'azote est fermée et le réacteur est clos. La réaction de polymérisation se déroule pendant 2 à 5 heures jusqu'à atteindre un pic de température. Le gel caoutchouteux obtenu est haché en particules d'une taille comprise entre 1 et 6 mm.

500,0 g de gel préalablement haché sont ensuite mélangés à 18,0 g de lessive de soude à 50%, le mélange est porté et maintenu à une température de 90°C pour une durée de 90 minutes.

Le gel est ensuite séché et broyé pour obtenir le polymère sous forme de poudre.

Exemple 20 : Préparation du copolymère P'3 acrylamide/acide 2-acrylamido-2-méthylpropane sulfonique de forme non cristalline hydratée (75/25 mole%) post hydrolysé

Le copolymère est préparé comme dans l'exemple 19 en remplaçant l'acide 2-acrylamido-2-méthylpropane sulfonique de forme cristalline hydratée (exemple 2) par de l'acide 2-acrylamido-2-méthylpropane sulfonique n'étant pas de forme cristalline hydratée obtenue en exemple 1.

## Revendications

1. Forme cristalline hydratée de l'acide 2-acrylamido-2-méthylpropane sulfonique ayant un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta(+/- 0.1°).

2. Forme cristalline hydratée de l'acide 2-acrylamido-2-méthylpropane sulfonique selon la revendication 1, ***caractérisée* en ce qu'**elle présente un spectre infra-rouge à transformée de Fourier comprenant des pics à $3280cm^{-1}$, $3126cm^{-1}$, $1657cm^{-1}$, $1595cm^{-1}$, $1453cm^{-1}$, $1395cm^{-1}$, $1307cm^{-1}$, $1205cm^{-1}$, $1164cm^{-1}$, $1113cm^{-1}$, $1041cm^{-1}$, $968cm^{-1}$, $885cm^{-1}$, $815cm^{-1}$, $794cm^{-1}$(+/- $8cm^{-1}$).

3. Forme cristalline hydratée de l'acide 2-acrylamido-2-méthylpropane sulfonique selon la revendication 1 ou 2, ***caractérisée* en ce qu'**elle présente une énergie minimale d'inflammation supérieure à 400 mJ, préférentiellement supérieure à 500 mJ.

4. Forme cristalline hydratée de l'acide 2-acrylamido-2-méthylpropane sulfonique selon l'une des revendications 1 à 3, ***caractérisée* en ce qu'**elle présente 4 phénomènes thermiques avec la technique de calorimétrie différentielle à balayage, à 70 °C, 100°C, 150°C et 190°C (+/-10°C).

5. Forme cristalline hydratée de l'acide 2-acrylamido-2-méthylpropane sulfonique selon l'une des revendications 1 à 4, ***caractérisée* en ce qu'**elle présente un rapport molaire eau/acide 2-acrylamido-2-méthylpropane sulfonique égal à 1.

6. Procédé de fabrication de la forme cristalline hydratée de l'acide 2-acrylamido-2-méthylpropane sulfonique selon l'une des revendications 1 à 5, comprenant au moins les étapes successives suivantes :

   1) mélange d'acide 2-acrylamido-2-méthylpropane sulfonique avec une solution aqueuse, avantageusement pendant au moins 1 minute, afin de former une suspension A,
   la solution aqueuse comprenant un solvant 1 organique et/ou un acide inorganique différent de l'acide 2-acrylamido-2-méthylpropane sulfonique,
   le solvant 1 organique étant linéaire ou ramifié et choisi parmi les composés suivants :

      - acides organiques, avantageusement les acides carboxyliques comprenant de 1 à 8 carbones,
      - les amides comprenant avantageusement de 1 à 8 atomes de carbone,
      - les alcools comprenant avantageusement de 1 à 8 atomes de carbones,
      - les cétones comprenant avantageusement de 1 à 8 atomes de carbones,
      - les éthers comprenant avantageusement de 1 à 8 atomes de carbones,
      - les esters comprenant avantageusement de 1 à 8 atomes de carbones,
      - les alcanes comprenant avantageusement de 1 à 8 atomes de carbones,
      - les composés hydrocarbonés halogénés comprenant avantageusement de 1 à 8 atomes de carbones,
      - les nitriles comprenant avantageusement de 1 à 8 atomes de carbones, ou
      - leurs mélanges.

   2) chauffage de la suspension A, à une température avantageusement comprise entre 40 et 150°C, afin d'obtenir une solution B d'acide 2-acrylamido-2-méthylpropane sulfonique,
   3) refroidissement de la solution B, avantageusement à une température comprise entre -40 et 100°C, avantageusement pendant une durée comprise entre 1 et 1200 minutes, afin d'obtenir une suspension C de cristaux,
4) séparation solide / liquide de la suspension C et isolement des cristaux de la suspension C obtenue à l'issue de l'étape 3) sous la forme d'une composition 1 dans laquelle les cristaux représentent avantageusement entre 40 et 99 % en poids de la composition 1.

**7.** Procédé selon la revendication 6, ***caractérisé* en ce qu'**en étape 1) le ratio en poids entre l'acide 2-acrylamido-2-méthylpropane sulfonique et la solution aqueuse est compris entre 0,1:1 et 5:1, préférentiellement entre 0,2:1 et 3:1.

**8.** Procédé selon l'une des revendications 6 à 7, ***caractérisé* en ce que** la solution aqueuse de l'étape 1) comprend au moins 80% en poids d'eau et jusqu'à 20% en poids de solvant 1 organique.

**9.** Procédé selon l'une des revendications 6 à 7, ***caractérisé* en ce que** la solution aqueuse de l'étape 1) comprend au moins 80% en poids d'eau et jusqu'à 20% en poids d'acide inorganique.

**10.** Procédé selon l'une des revendications 6 à 9, ***caractérisé* en ce que**, lors de l'étape 2), la suspension A obtenue à l'étape 1) est chauffée à une température comprise entre 50 et 120°C, afin d'obtenir une solution B.

**11.** Procédé selon l'une des revendications 6 à 10, **caractérisé en ce que**, lors de l'étape 3), la solution B obtenue à l'étape 2) est refroidie à une température comprise entre - 20 et 50°C.

**12.** Procédé selon l'une des revendications 6 à 11, ***caractérisé* en ce que** l'étape 4) est réalisée à une température comprise entre -40 et 100°C, préférentiellement entre -20 et 50°C.

**13.** Procédé selon l'une des revendications 6 à 12, ***caractérisé* en ce qu'**après l'étape 4) les cristaux d'acide 2-acrylamido-2-méthylpropane sulfonique ne sont pas séchés.

**14.** Procédé de préparation d'une solution aqueuse A de sel de l'acide 2-acrylamido-2-méthylpropane sulfonique selon l'une des revendications 1 à 5, comprenant les étapes suivantes :

a) préparation d'une solution aqueuse X d'acide 2-acrylamido-2-méthylpropane sulfonique selon la revendication 1, avantageusement de concentration comprise entre 1 et 700 g/L,
b) mise en contact et mélange de la solution aqueuse X avec un composé Y choisi parmi un hydroxyde de métal alcalin ou alcalino-terreux, un oxyde de métal alcalin ou alcalino-terreux, l'ammoniaque, une amine de formule suivante $NR_1R_2R_3$ ou un carbonate de métal alcalin ou alcalino-terreux.

**15.** Procédé selon la revendication 14, ***caractérisé* en ce que** le ratio molaire entre l'acide 2-acrylamido-2-méthylpropane sulfonique et le composé Y est compris entre 1:0,1 et 1:1,1, préférentiellement entre 1:0,5 et 1:1,05.

**16.** (Co)polymère obtenu à partir au moins d'acide 2-acrylamido-2-méthylpropane sulfonique, sous sa forme acide et/ou salifiée,
au moins une partie de l'acide 2-acrylamido-2-méthylpropane sulfonique étant sous forme cristalline hydratée et ayant un diagramme de diffraction des rayons X sur poudre comprenant des pics à 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04°degrés 2-thêta(+/- 0.1°).

**17.** (Co)polymère selon la revendication 16, ***caractérisé* en ce que** le (co)polymère est obtenu, en outre, à partir d'au moins un monomère non ionique et/ou au moins un monomère cationique et/ou un monomère zwittérionique et/ou au moins un monomère anionique distinct de l'acide 2-acrylamido-2-méthylpropane sulfonique étant sous forme cristalline hydratée.

**Patentansprüche**

**1.** Hydratisierte kristalline Form von 2-Acrylamido-2-methylpropansulfonsäure mit einem 2-Theta-Pulver-Röntgenbeugungsdiagramm, das Peaks bei Graden (+/- 0,1°) von 10,58°, 11,2°, 12,65°, 13,66°, 16,28°, 18,45°, 20°, 20,4°, 22,5°, 25,5°, 25,88°, 26,47°, 28,52°, 30,28°, 30,8°, 34,09°, 38,19°, 40,69°, 41,82°, 43,74°, 46,04° aufweist.

**2.** Hydratisierte kristalline Form von 2-Acrylamido-2-methylpropansulfonsäure gemäß Anspruch 1, **dadurch gekenn-**

**zeichnet, dass** sie ein Fourier-Transformations-InfrarotSpektrum zeigt, das Peaks bei 3280 $cm^{-1}$, 3126 $cm^{-1}$, 1657 $cm^{-1}$, 1595 $cm^{-1}$, 1453 $cm^{-1}$, 1395 $cm^{-1}$, 1307 $cm^{-1}$, 1205 $cm^{-1}$, 1164 $cm^{-1}$, 1113 $cm^{-1}$, 1041 $cm^{-1}$, 968 $cm^{-1}$, 885 $cm^{-1}$, 815 $cm^{-1}$, 794 $cm^{-1}$ (+/- 8 $cm^{-1}$) aufweist.

**3.** Hydratisierte kristalline Form von 2-Acrylamido-2-methylpropansulfonsäure gemäß Anspruch 1 oder 2, **dadurch gekennzeichnet, dass** sie eine Mindestzündenergie von mehr als 400 mJ, vorzugsweise mehr als 500 mJ, aufweist.

**4.** Hydratisierte kristalline Form von 2-Acrylamido-2-methylpropansulfonsäure gemäß einem der Ansprüche 1 bis 3, **dadurch gekennzeichnet, dass** sie bei der Technik der dynamischen Differenzkalorimetrie 4 thermische Phänomene bei 70°C, 100°C, 150°C und 190°C (+/- 10°C) zeigt.

**5.** Hydratisierte kristalline Form von 2-Acrylamido-2-methylpropansulfonsäure gemäß einem der Ansprüche 1 bis 4, **dadurch gekennzeichnet, dass** sie ein Wasser/2-Acrylamido-2-methylpropansulfonsäure-Molverhältnis von 1 aufweist.

**6.** Verfahren zur Herstellung der hydratisierten kristallinen Form von 2-Acrylamido-2-methylpropansulfonsäure gemäß einem der Ansprüche 1 bis 5, welches zumindest die folgenden aufeinanderfolgenden Schritte umfasst:

1) das Mischen von 2-Acrylamido-2-methylpropansulfonsäure mit einer wässrigen Lösung vorteilhafterweise für mindestens 1 Minute, um Suspension A zu bilden, wobei die wässrige Lösung ein organisches Lösungsmittel 1 und/oder mindestens eine andere anorganische Säure als 2-Acrylamido-2-methylpropansulfonsäure umfasst, wobei das organische Lösungsmittel 1 linear oder verzweigt und aus den folgenden Verbindungen ausgewählt ist:

- organische Säuren, vorteilhafterweise Carbonsäuren mit 1 bis 8 Kohlenstoffen,
- Amide mit vorteilhafterweise 1 bis 8 Kohlenstoffatomen,
- Alkohole mit vorteilhafterweise 1 bis 8 Kohlenstoffatomen,
- Ketone mit vorteilhafterweise 1 bis 8 Kohlenstoffatomen,
- Ether mit vorteilhafterweise 1 bis 8 Kohlenstoffatomen,
- Ester mit vorteilhafterweise 1 bis 8 Kohlenstoffatomen,
- Alkane mit vorteilhafterweise 1 bis 8 Kohlenstoffatomen,
- halogenierte Kohlenwasserstoffverbindungen mit vorteilhafterweise 1 bis 8 Kohlenstoffatomen,
- Nitrile mit vorteilhafterweise 1 bis 8 Kohlenstoffatomen oder
- deren Gemische

2) das Erwärmen der Suspension A vorteilhafterweise auf eine Temperatur zwischen 40 und 150°C, um eine Lösung B von 2-Acrylamido-2-methylpropansulfonsäure herzustellen,
3) das Abkühlen der Lösung B vorteilhafterweise auf eine Temperatur zwischen -40 und 100°C, vorteilhafterweise für einen Zeitraum zwischen 1 und 1200 Minuten, um eine Suspension C von Kristallen herzustellen,
4) eine Fest-Flüssig-Trennung von Suspension C und das Isolieren von Kristallen aus der Suspension C aus Schritt 3) in Form einer Zusammensetzung 1, in der die Kristalle vorteilhafterweise zwischen 40 und 99 Gew.-% der Zusammensetzung 1 ausmachen.

**7.** Verfahren gemäß Anspruch 6, **dadurch gekennzeichnet, dass** in Schritt 1) das Gewichtsverhältnis zwischen 2-Acrylamido-2-methylpropansulfonsäure und der wässrigen Lösung im Bereich zwischen 0,1:1 und 5:1, vorzugsweise zwischen 0,2:1 und 3:1, liegt.

**8.** Verfahren gemäß einem der Ansprüche 6 bis 7, **dadurch gekennzeichnet, dass** die wässrige Lösung von Schritt 1) mindestens 80 Gew.-% Wasser und bis zu 20 Gew.-% organisches Lösungsmittel 1 umfasst.

**9.** Verfahren gemäß einem der Ansprüche 6 bis 7, **dadurch gekennzeichnet, dass** die wässrige Lösung von Schritt 1) mindestens 80 Gew.-% Wasser und bis zu 20 Gew.-% anorganische Säure umfasst.

**10.** Verfahren gemäß einem der Ansprüche 6 bis 9, **dadurch gekennzeichnet, dass** während Schritt 2) die in Schritt 1) gewonnene Suspension A auf eine Temperatur zwischen 50 und 120°C erhitzt wird, um eine Lösung B herzustellen.

**11.** Verfahren gemäß einem der Ansprüche 6 bis 10, **dadurch gekennzeichnet, dass** während Schritt 3) die in Schritt

2) gewonnene Suspension B auf eine Temperatur zwischen -20 und 50°C abgekühlt wird.

**12.** Verfahren gemäß einem der Ansprüche 6 bis 11, **dadurch gekennzeichnet, dass** Schritt 4) bei einer Temperatur zwischen -40 und 100°C, vorzugsweise zwischen -20 und 50°C, ausgeführt wird.

**13.** Verfahren gemäß einem der Ansprüche 6 bis 12, **dadurch gekennzeichnet, dass** die Kristalle der 2-Acrylamido-2-methylpropansulfonsäure nach Schritt 4) nicht getrocknet werden.

**14.** Verfahren zur Herstellung einer wässrigen Lösung A eines 2-Acrylamido-2-methylpropansulfonsäure-Salzes gemäß einem der Ansprüche 1 bis 5, umfassend die folgenden Schritte:

a) das Herstellen einer wässrigen Lösung X von 2-Acrylamido-2-methylpropansulfonsäure gemäß Anspruch 1, vorzugsweise mit einer Konzentration zwischen 1 und 700 g/l;
b) das Inkontaktbringen und Mischen der wässrigen Lösung X mit einer Verbindung Y, ausgewählt aus einem Alkali- oder Erdalkalimetallhydroxid, einem Alkali- oder Erdalkalimetalloxid, Ammoniak, einem Amin mit der folgenden Formel $NR_1R_2R_3$ oder einem Alkali- oder Erdalkalimetallcarbonat.

**15.** Verfahren gemäß Anspruch 14, **dadurch gekennzeichnet, dass** das Molverhältnis zwischen 2-Acrylamido-2-methylpropansulfonsäure und der Verbindung Y im Bereich zwischen 1:0,1 und 1:1,1, vorzugsweise zwischen 1:0,5 und 1:1,05, liegt.

**16.** Zumindest aus 2-Acrylamido-2-methylpropansulfonsäure gewonnenes (Co-)Polymer in seiner sauren Form und/oder Salzform, wobei zumindest ein Teil der 2-Acrylamido-2-methylpropansulfonsäure in hydratisierter kristalliner Form vorliegt und ein Pulver-Röntgenbeugungsmuster mit Peaks bei 2-Theta-Graden (+/- 0,1°) von 10,58°, 11,2°, 12,65°, 13,66°, 16,28°, 18,45°, 20°, 20,4°, 22,5°, 25,5°, 25,88°, 26,47°, 28,52°, 30,28°, 30,8°, 34,09°, 38,19°, 40,69°, 41,82°, 43,74°, 46,04° aufweist.

**17.** (Co-)Polymer gemäß Anspruch 16, **dadurch gekennzeichnet, dass** das (Co-)Polymer zusätzlich aus mindestens einem nichtionischen Monomer und/oder mindestens einem kationischen Monomer und/oder einem zwitterionischen Monomer und/oder mindestens einem anionischen Monomer gewonnen ist, das sich von der 2-Acrylamido-2-methylpropansulfonsäure unterscheidet, die in hydratisierter kristalliner Form vorliegt.

## Claims

**1.** A hydrated crystalline form of 2-acrylamido-2-methylpropane sulfonic acid having a 2-theta powder X-ray diffraction diagram comprising peaks at 10.58°, 11.2°, 12.65°, 13.66°, 16.28°, 18.45°, 20°, 20.4°, 22.5°, 25.5°, 25.88°, 26.47°, 28.52°, 30.28°, 30.8°, 34.09°, 38.19°, 40.69°, 41.82°, 43.74°, 46.04° degrees (+/- 0.1°).

**2.** A hydrated crystalline form of 2-acrylamido-2-methylpropane sulfonic acid according to claim 1, ***characterized* in that** it presents a Fourier transform infrared spectrum comprising peaks at 3280 $cm^{-1}$, 3126 $cm^{-1}$, 1657 $cm^{-1}$, 1595 $cm^{-1}$, 1453 $cm^{-1}$, 1395 $cm^{-1}$, 1307 $cm^{-1}$, 1205 $cm^{-1}$, 1164 $cm^{-1}$, 1113 $cm^{-1}$, 1041 $cm^{-1}$, 968 $cm^{-1}$, 885 $cm^{-1}$, 815 $cm^{-1}$, 794 $cm^{-1}$ (+/- 8 $cm^{-1}$).

**3.** The hydrated crystalline form of 2-acrylamido-2-methylpropane sulfonic acid according to claim 1 or 2, ***characterized* in that** it presents minimum ignition energy greater than 400 mJ, preferably greater than 500 mJ.

**4.** The hydrated crystalline form of 2-acrylamido-2-methylpropane sulfonic acid according to one of claims 1 to 3, ***characterized* in that** it presents 4 thermal phenomena with the Differential Scanning Calorimetry technique at 70°C, 100°C, 150°C and 190°C (+/-10°C).

**5.** The hydrated crystalline form of 2-acrylamido-2-methylpropane sulfonic acid according to one of claims 1 to 4, ***characterized* in that** it has a water/2-acrylamido-2-methylpropane sulfonic acid molar ratio of 1.

**6.** A method of manufacturing the hydrated crystalline form of 2-acrylamido-2-methylpropane sulfonic acid according to one of claims 1 to 5, comprising at least the following successive steps:

1) Mix 2-acrylamido-2-methylpropane sulfonic acid with an aqueous solution, advantageously for at least 1

minute, to form suspension A, the aqueous solution comprising an organic solvent 1 and/or at least an inorganic acid other than 2-acrylamido-2-methylpropane sulfonic acid, the organic solvent 1 being linear or branched and chosen from the following compounds:

- organic acids, advantageously carboxylic acids comprising from 1 to 8 carbons,
- amides comprising advantageously from 1 to 8 carbon atoms,
- alcohols comprising advantageously from 1 to 8 carbon atoms,
- ketones comprising advantageously from 1 to 8 carbon atoms,
- ethers comprising advantageously from 1 to 8 carbon atoms,
- esters comprising advantageously from 1 to 8 carbon atoms,
- alkanes comprising advantageously from 1 to 8 carbon atoms,
- halogenated hydrocarbon compounds comprising advantageously from 1 to 8 carbon atoms,
- nitriles comprising advantageously from 1 to 8 carbon atoms, or
- their mixtures.

2) Heat suspension A, advantageously to a temperature of between 40 and 150°C, to produce a solution B of 2-acrylamido-2-methylpropane sulfonic acid,
3) Cool solution B, advantageously to a temperature of between -40 and 100°C, advantageously for a period of between 1 and 1200 minutes, to produce a suspension C of crystals,
4) Solid/liquid separation from suspension C and isolate crystals from suspension C from step 3) in the form of a composition 1 in which the crystals advantageously represent between 40 and 99% by weight of composition 1.

**7.** The method according to claim 6, ***characterized* in that** in step 1) the ratio by weight between 2-acrylamido-2-methylpropane sulfonic acid and the aqueous solution is between 0.1:1 and 5:1, preferably between 0.2:1 and 3:1.

**8.** The method according to one of claims 6 to 7 ***characterized* in that** the aqueous solution of step 1) comprises at least 80% by weight and up to 20% by weight of organic solvent 1.

**9.** The method according to one of claims 6 to 7 ***characterized* in that** the aqueous solution of step 1) comprises at least 80% by weight and up to 20% by weight of inorganic acid.

**10.** The method according to one of claims 6 to 9, ***characterized* in that** during step 2), suspension A obtained in step 1) is heated to a temperature of between 50 and 120°C, to produce solution B.

**11.** The method according to one of claims 6 to 10, ***characterized* in that** during step 3), suspension B obtained in step 2) is cooled to a temperature of between -20 and 50°C.

**12.** The method according to one of claims 6 to 11, ***characterized* in that** step 4) is conducted at a temperature of between -40 and 100°C, preferably between - 20 et 50°C.

**13.** The method according to one of claims 6 to 12, ***characterized* in that** after step 4) the crystals of 2-acrylamido-2-methylpropane sulfonic acid are not dried.

**14.** The preparation method of an aqueous solution A of a 2-acrylamido-2-methylpropane sulfonic acid salt according to one of claims 1 to 5, comprising the following steps:

a) Preparing an aqueous solution X of 2-acrylamido-2-methylpropane sulfonic acid according to claim 1, preferably having a concentration between 1 and 700 g/L,
b) Putting in contact and mixing the aqueous solution X with a compound Y chosen from an alkali or alkaline earth metal hydroxide, an alkali or alkaline earth metal oxide, ammonia, an amine having the following formula $NR_1R_2R_3$ or an alkali or alkaline earth metal carbonate.

**15.** The method according to claim 14, ***characterized* in that** the molar ratio between 2-acrylamido-2-methylpropane sulfonic acid and compound Y is between 1:0.1 and 1:1.1, preferably between 1:0.5 and 1:1.05.

**16.** (Co)polymer obtained from at least 2-acrylamido-2-methylpropanesulfonic acid, in its acidic and/or salified form, at least a portion of the 2-acrylamido-2-methylpropanesulfonic acid being in hydrated crystalline form and having a powder X-ray diffraction pattern including peaks at 10.58°, 11.2°, 12.65°, 13.66°, 16.28° , 18.45°, 20°, 20.4°, 22.5°,

25.5°, 25.88°, 26.47°, 28.52°, 30.28°, 30.8°, 34.09°, 38.19°, 40.69°, 41.82°, 43.74°, 46.04° degrees 2 -theta (+/- 0.1°).

**17.** (Co)polymer according to claim 16, ***characterized*** **in that** the (co)polymer is obtained, in addition, from at least one non-ionic monomer and/or at least one cationic monomer and/or a zwitterionic monomer and/or at least one anionic monomer distinct from 2-acrylamido-2-methylpropanesulfonic acid being in hydrated crystalline form.

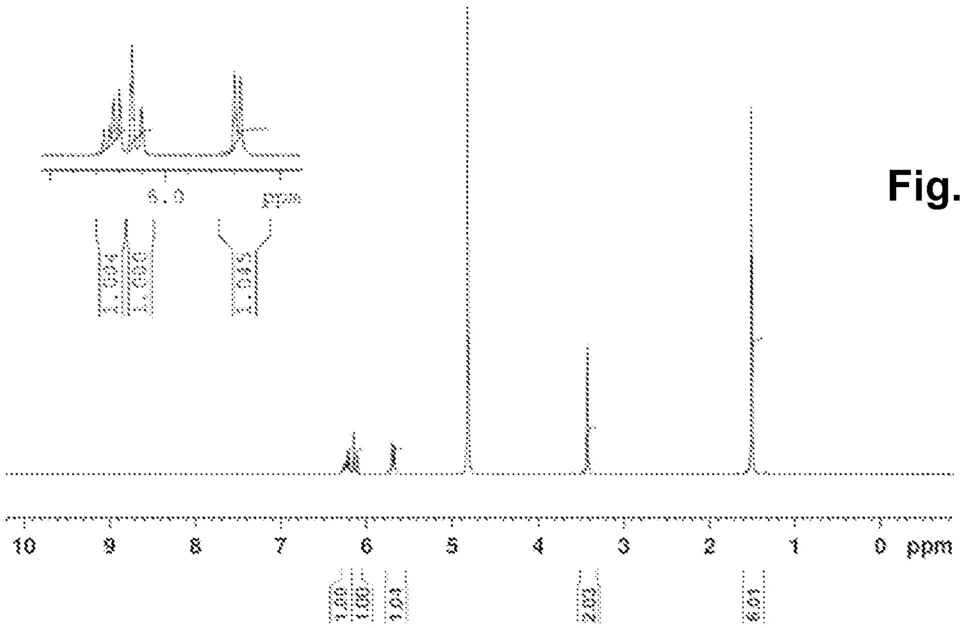


Fig. 1

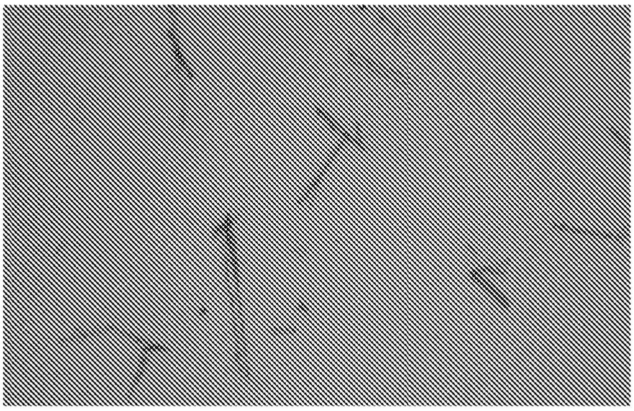

Fig. 10

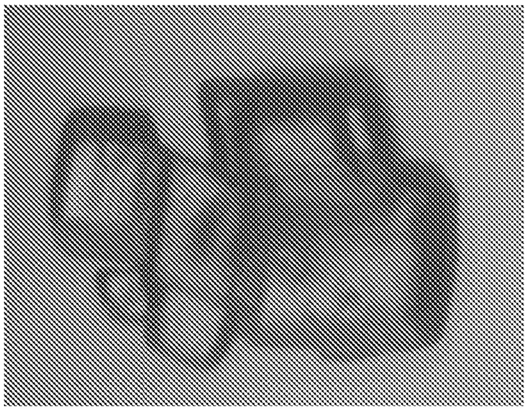

Fig. 11

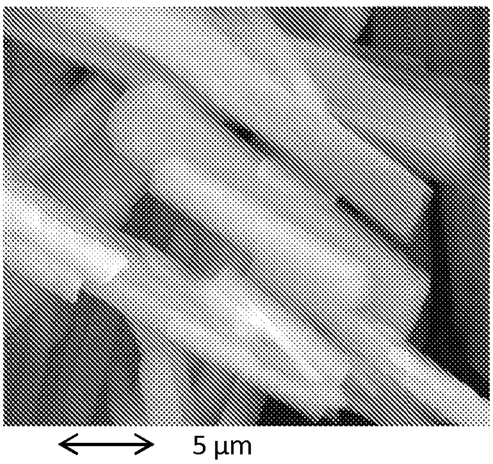

5 µm

Fig. 12

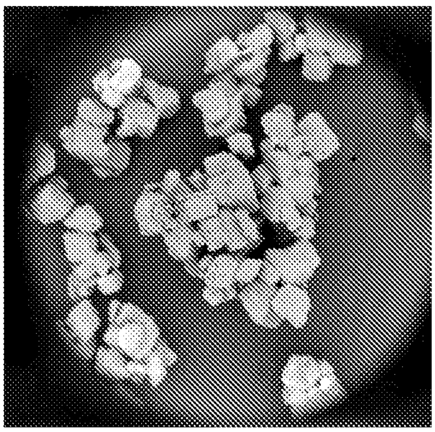

500 µm

Fig. 13

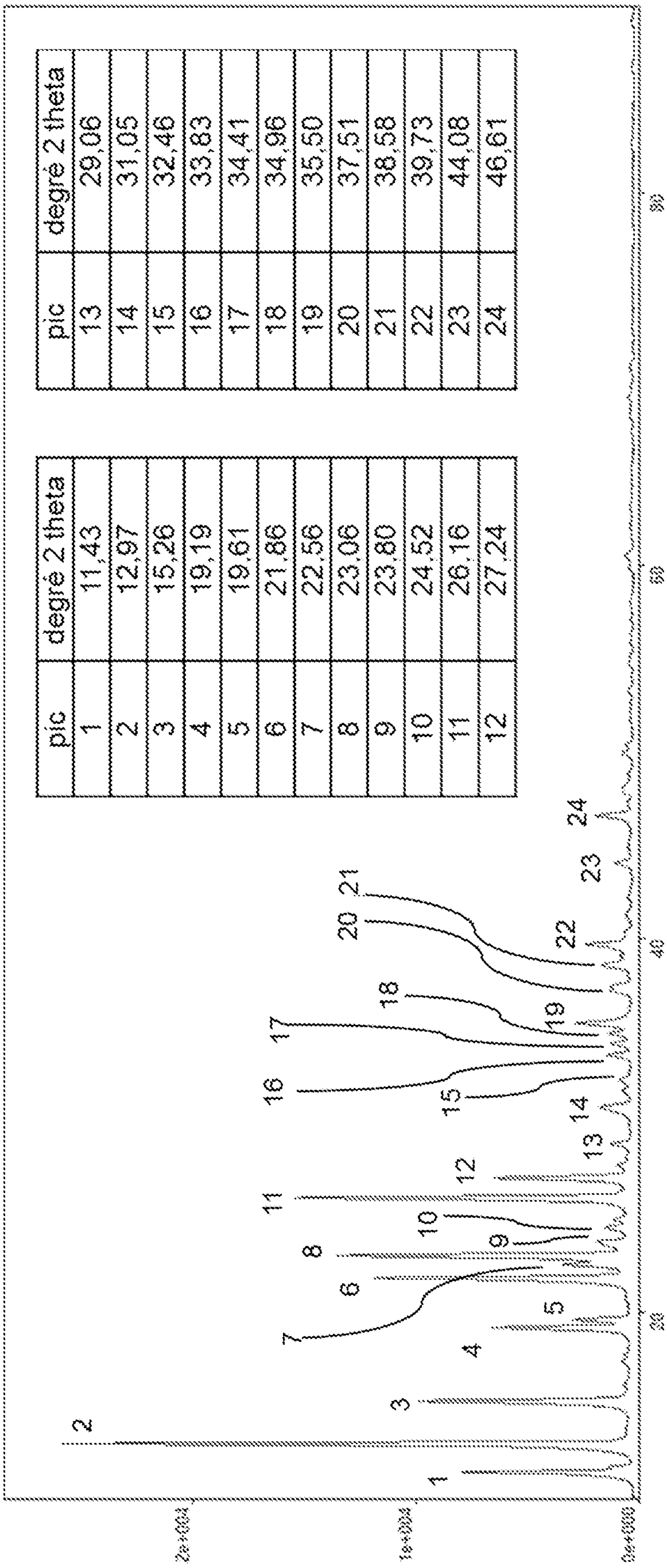


| pic | degré 2 theta | pic | degré 2 theta |
|---|---|---|---|
| 1 | 11,43 | 13 | 29,06 |
| 2 | 12,97 | 14 | 31,05 |
| 3 | 15,26 | 15 | 32,46 |
| 4 | 19,19 | 16 | 33,83 |
| 5 | 19,61 | 17 | 34,41 |
| 6 | 21,86 | 18 | 34,96 |
| 7 | 22,56 | 19 | 35,50 |
| 8 | 23,06 | 20 | 37,51 |
| 9 | 23,80 | 21 | 38,58 |
| 10 | 24,52 | 22 | 39,73 |
| 11 | 26,16 | 23 | 44,08 |
| 12 | 27,24 | 24 | 46,61 |

FIG. 2

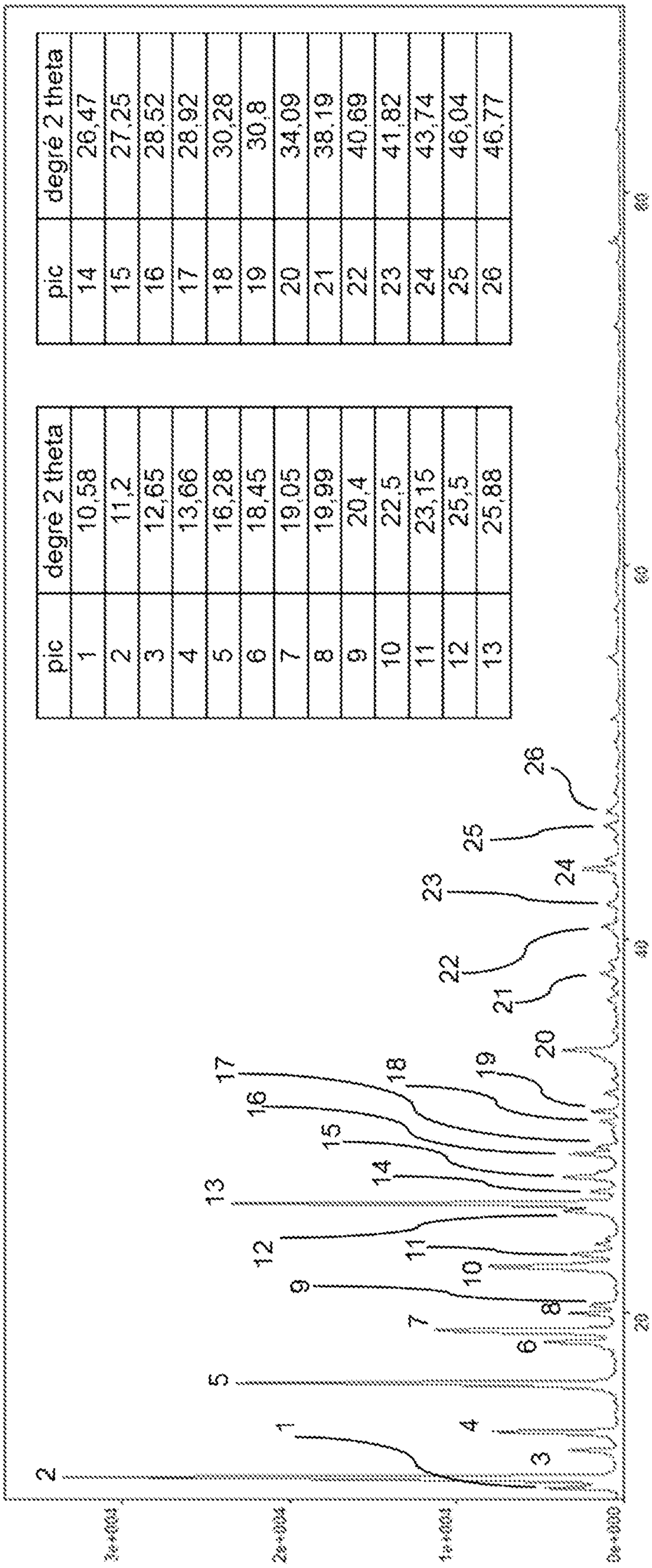

| pic | degré 2 theta |
|---|---|
| 1 | 10,58 |
| 2 | 11,2 |
| 3 | 12,65 |
| 4 | 13,66 |
| 5 | 16,28 |
| 6 | 18,45 |
| 7 | 19,05 |
| 8 | 19,99 |
| 9 | 20,4 |
| 10 | 22,5 |
| 11 | 23,15 |
| 12 | 25,5 |
| 13 | 25,88 |

| pic | degré 2 theta |
|---|---|
| 14 | 26,47 |
| 15 | 27,25 |
| 16 | 28,52 |
| 17 | 28,92 |
| 18 | 30,28 |
| 19 | 30,8 |
| 20 | 34,09 |
| 21 | 38,19 |
| 22 | 40,69 |
| 23 | 41,82 |
| 24 | 43,74 |
| 25 | 46,04 |
| 26 | 46,77 |

FIG. 3

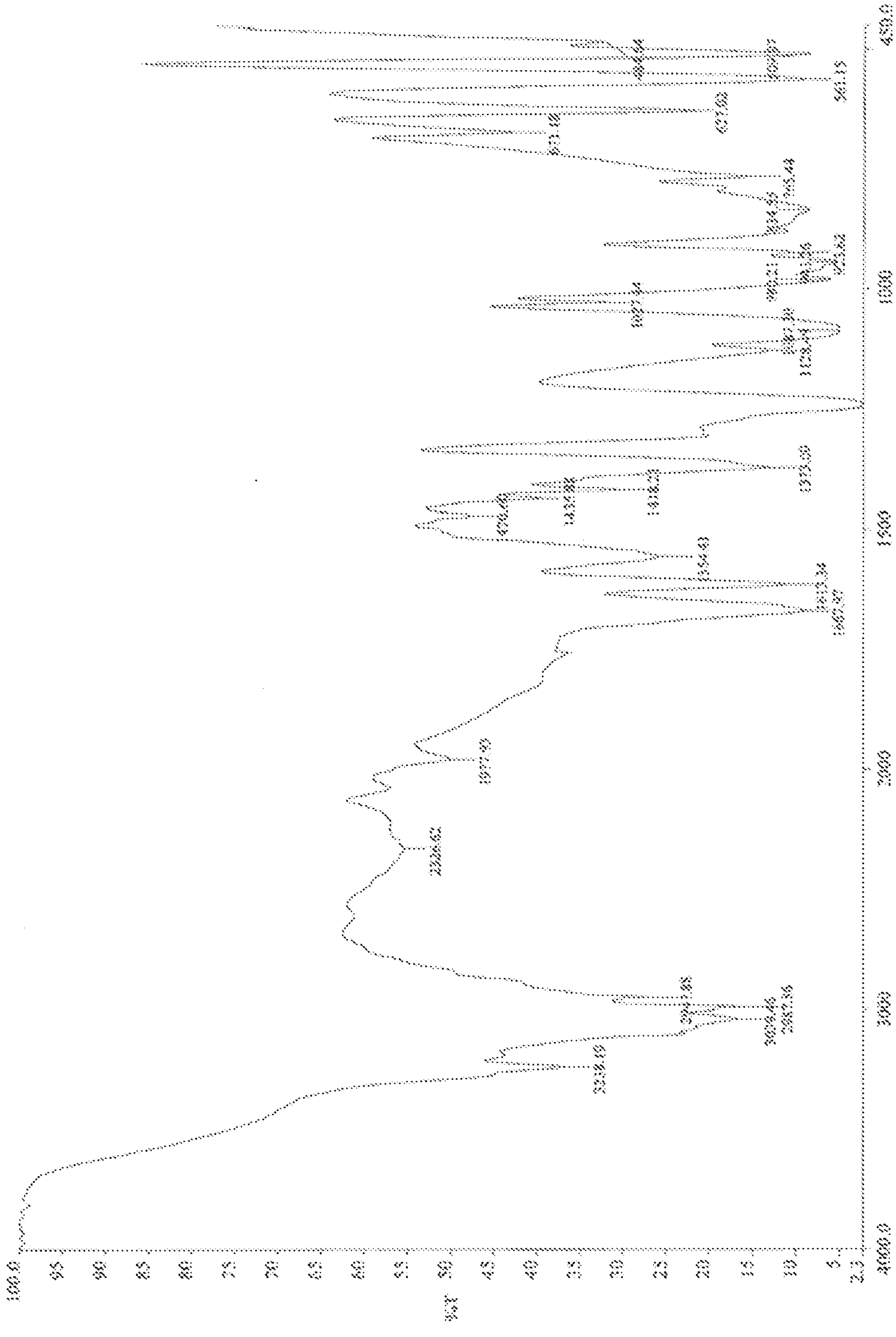

FIG. 4

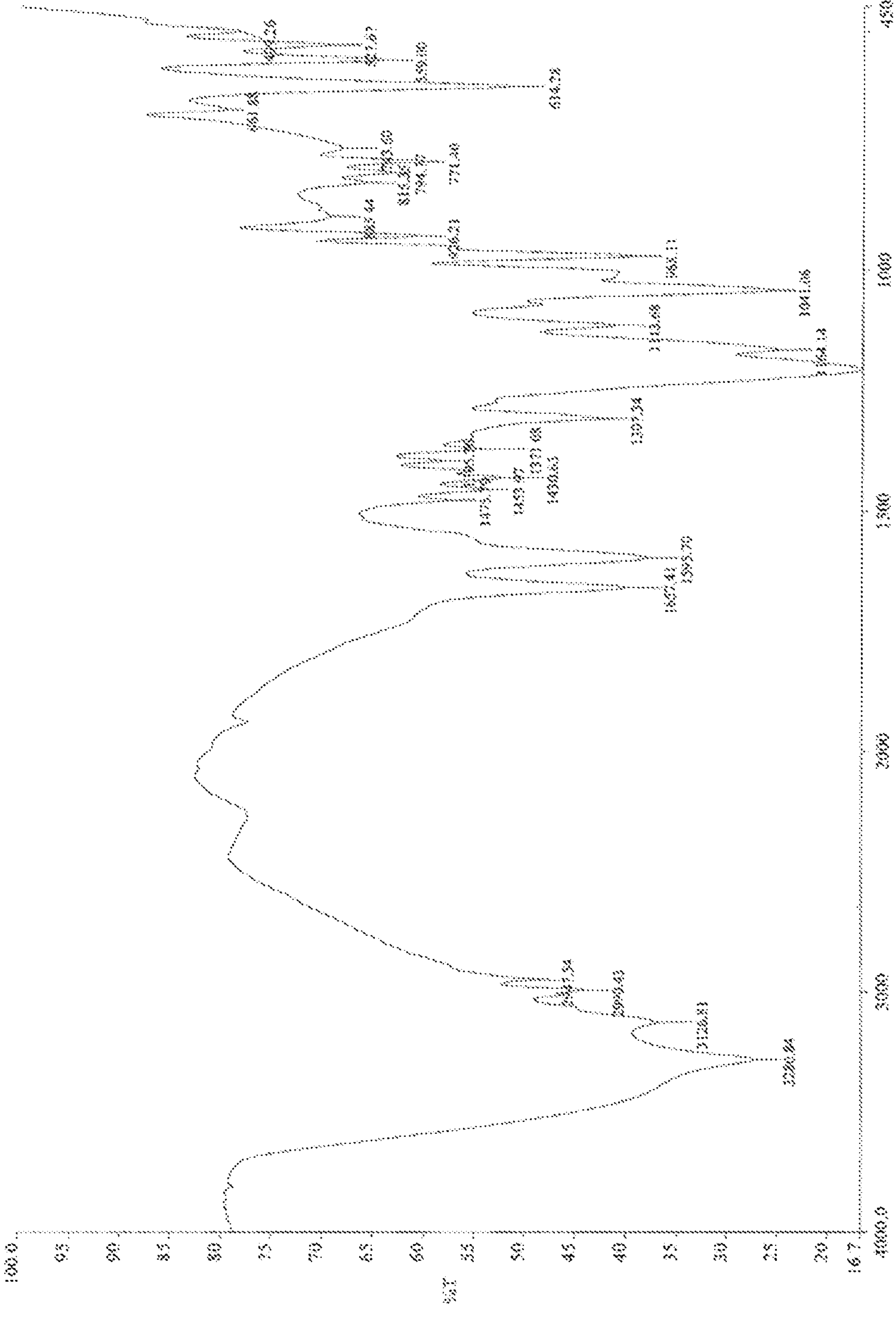

FIG. 5

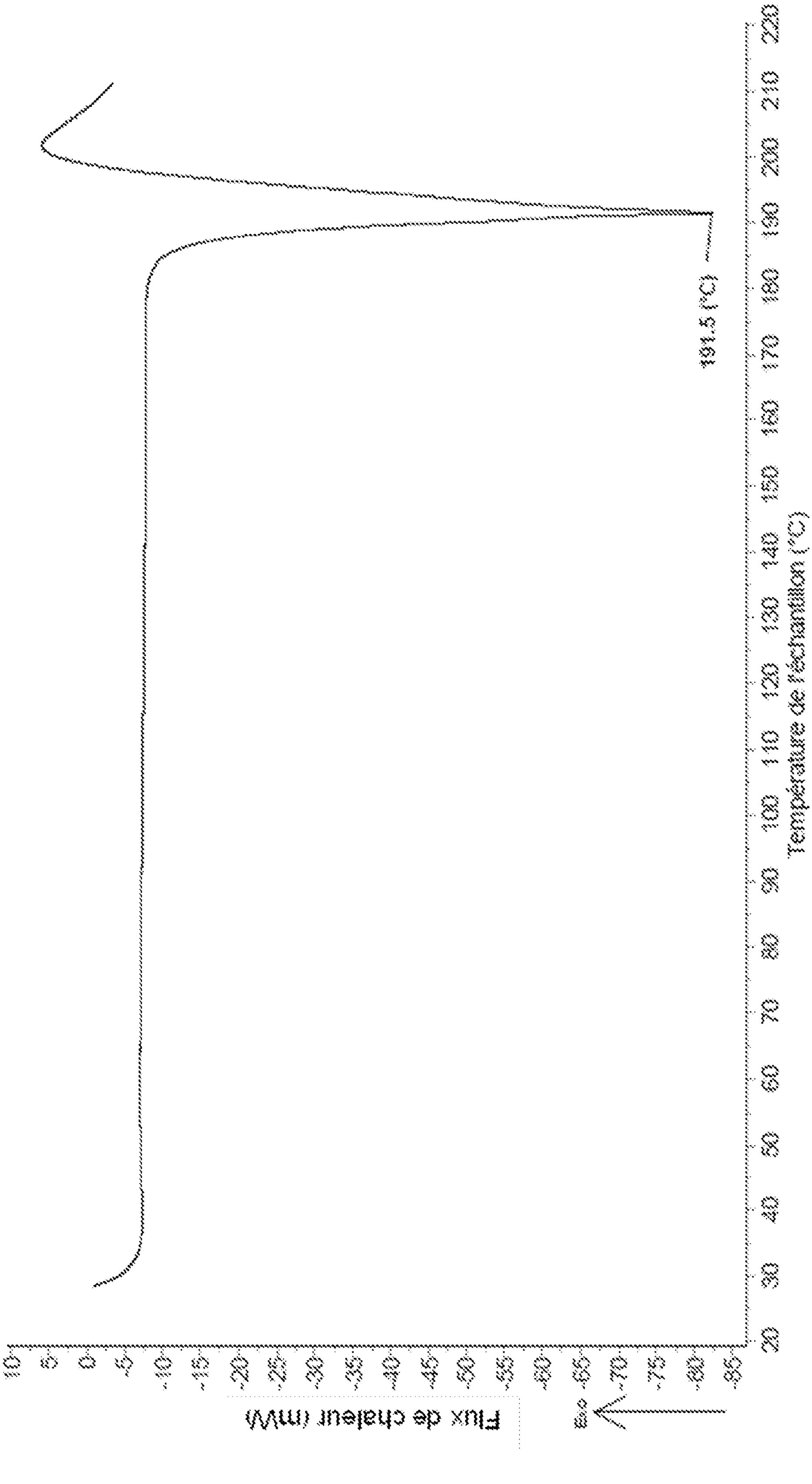


FIG. 6

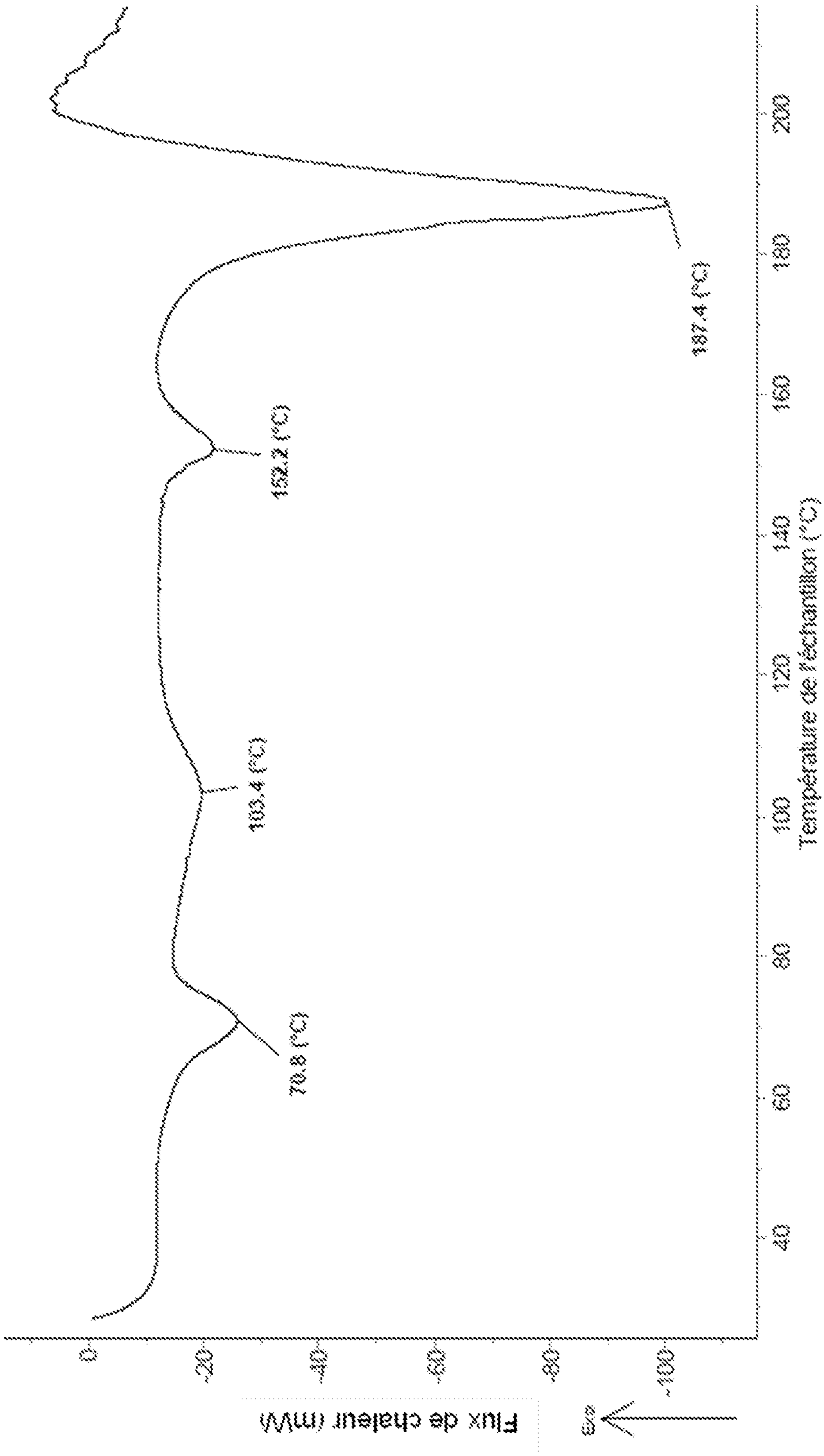
Flux de chaleur (mW)
Exo
0
-20
-40
-60
-80
-100
40
60
80
100
120
140
160
180
200
Température de l'échantillon (°C)
78.8 (°C)
103.4 (°C)
152.2 (°C)
187.4 (°C)

FIG. 7

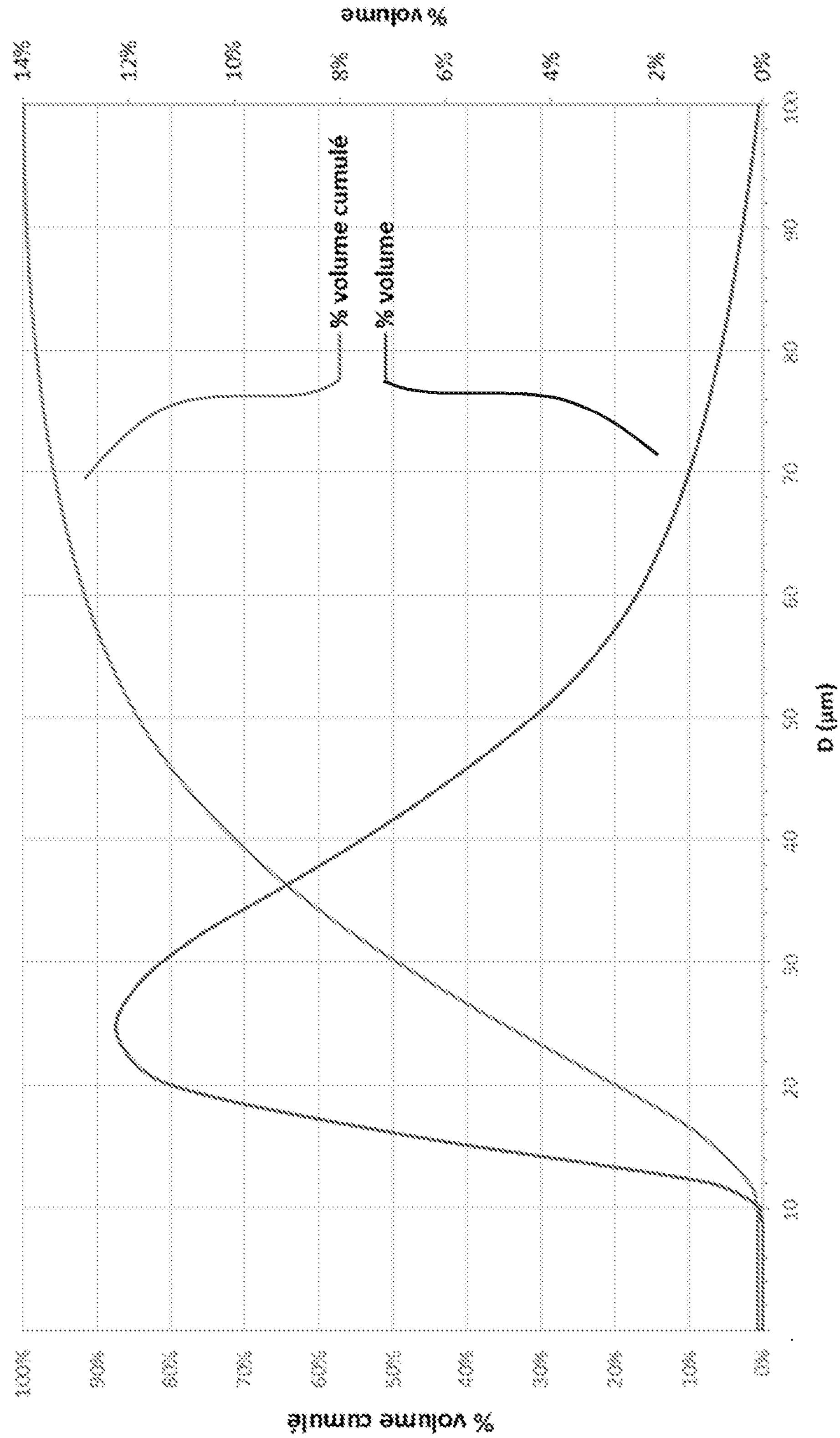
% volume cumulé
% volume
D (µm)
% volume cumulé
% volume
0%
2%
4%
6%
8%
10%
12%
14%
0%
10%
20%
30%
40%
50%
60%
70%
80%
90%
100%
10
20
30
40
50
60
70
80
90
100

FIG. 8

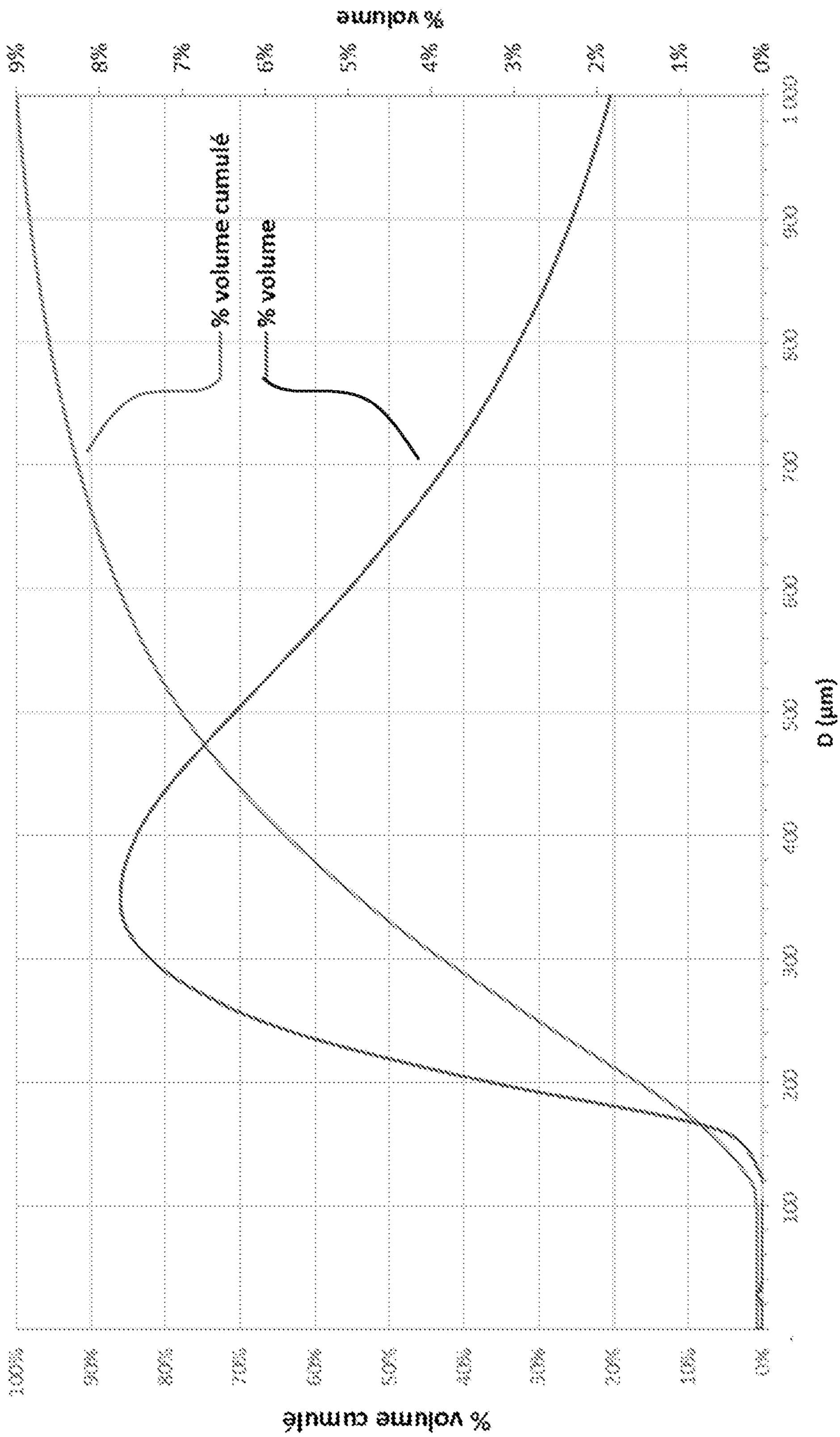
% volume
9%
8%
7%
6%
5%
4%
3%
2%
1%
0%
% volume cumulé
% volume
D (µm)
% volume cumulé
100%
90%
80%
70%
60%
50%
40%
30%
20%
10%
0%

FIG. 9

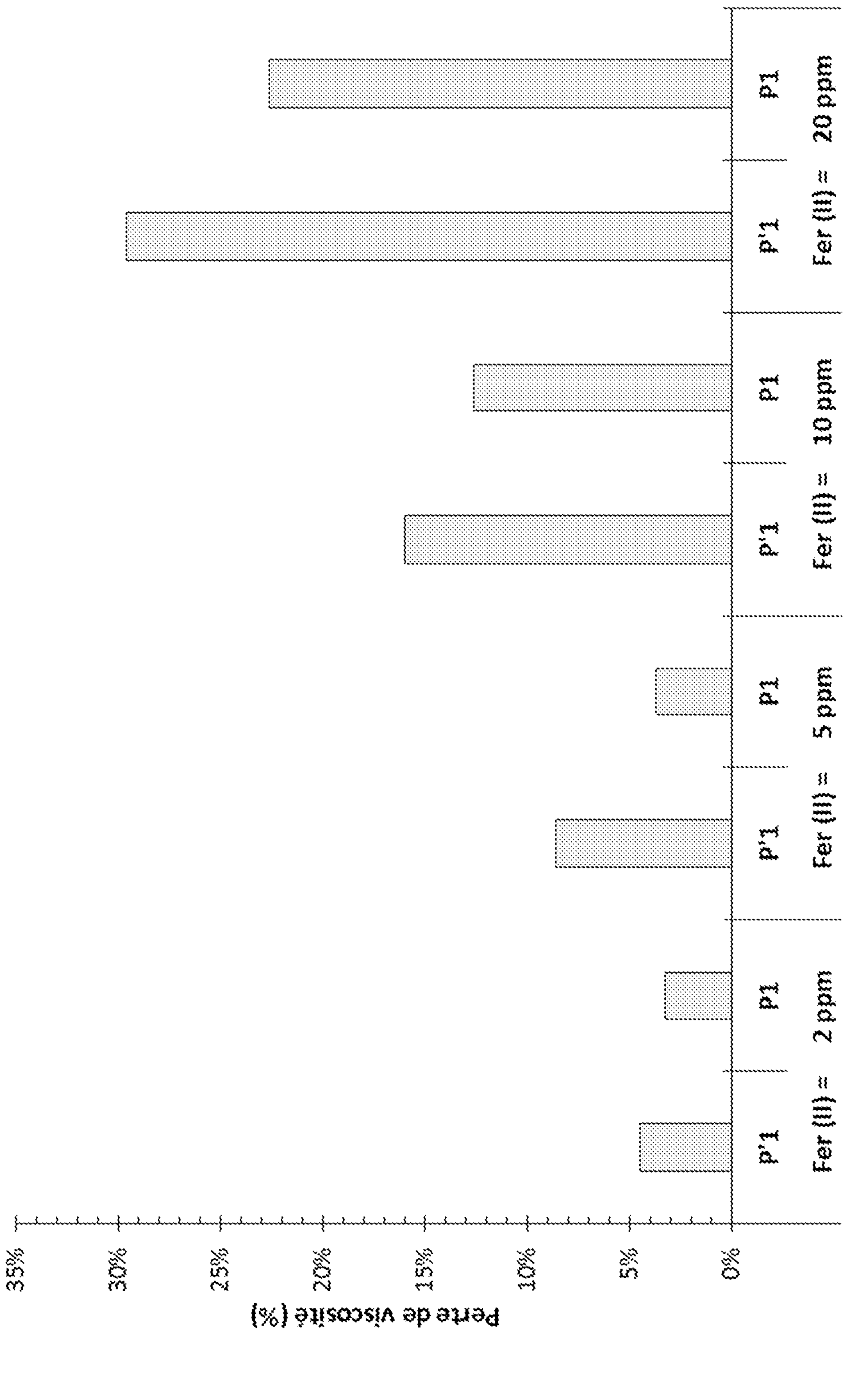
Perte de viscosité (%)
0%
5%
10%
15%
20%
25%
30%
35%
P'1
P1
Fer (II) = 2 ppm
P'1
P1
Fer (II) = 5 ppm
P'1
P1
Fer (II) = 10 ppm
P'1
P1
Fer (II) = 20 ppm

FIG. 14

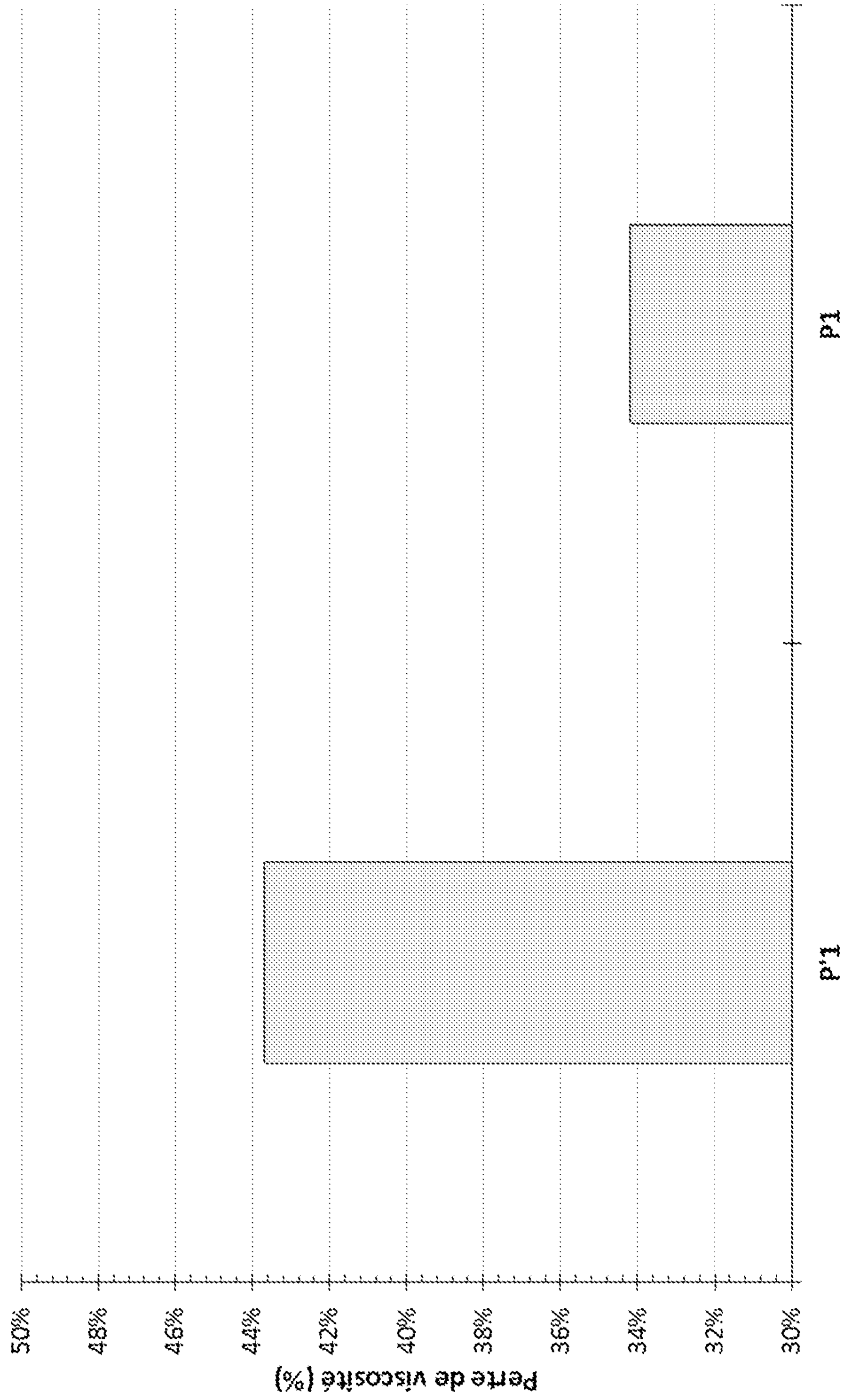


FIG. 15

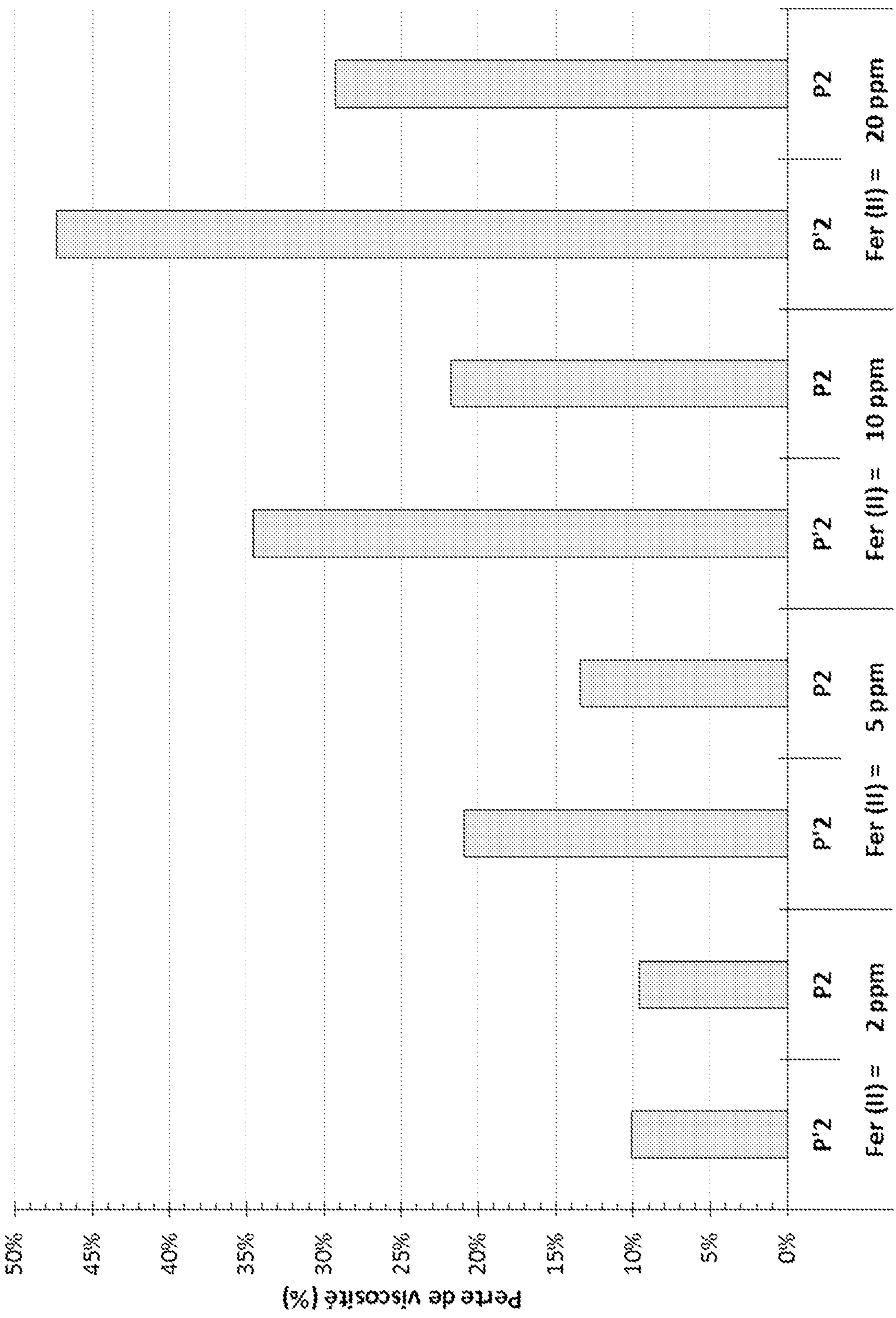
Perte de viscosité (%)
0%
5%
10%
15%
20%
25%
30%
35%
40%
45%
50%
P'2
P2
Fer (III) = 2 ppm
P'2
P2
Fer (III) = 5 ppm
P'2
P2
Fer (III) = 10 ppm
P'2
P2
Fer (III) = 20 ppm

FIG. 16

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6448347 B
- CN 102351744
- WO 2009072480 A
- JP 2008307822 A
- JP 2003137857 A